# EUROPÄISCHE PATENTSCHRIFT

(11) **EP 4 074 684 B1**
(45) Veröffentlichungstag und Bekanntmachung des Hinweises auf die Patenterteilung: **03.09.2025**
(21) Anmeldenummer: 21168921.1
(22) Anmeldetag: 16.04.2021
(51) Int. Cl.: C07C 29/70, C07C 31/30, C07C 29/80, C07C 31/04

(54) **VERFAHREN ZUR ENERGIEEFFIZIENTEN HERSTELLUNG VON ALKALIMETALLALKOHOLATEN**
METHOD FOR THE ENERGY-EFFICIENT PRODUCTION OF ALKALI METAL ETHANOLATES
PROCÉDÉ DE PRODUCTION ÉCONOMIQUE EN ÉNERGIE D'ALCOOLATES ALCALIMÉTALES

(43) Veröffentlichungstag der Anmeldung: 19.10.2022
(73) Patentinhaber: Evonik Operations GmbH, 45128 Essen (DE)
(72) Erfinder: ROETTGER, Dirk, 50672 Köln (DE); REIMANN, Sebastian, 50389 Wesseling (DE); RIX, Armin Matthias, 45770 Marl (DE); PAUL, Niklas, 45770 Marl (DE); SCHRÖDER, Moritz, 48153 Münster (DE); ZITZEWITZ, Philip, 45721 Haltern am See (DE)
(74) Vertreter: Evonik Patent Association

(56) Entgegenhaltungen:
- WO-A1-2010/097318
- CN-A- 109 627 145

## Beschreibung

Die vorliegende Erfindung betrifft ein Verfahren zur Herstellung von Natrium- und/oder Kaliumalkoholaten im Gegenstrom durch Reaktivrektifikation. Dabei wird Alkohol im Gegenstrom mit dem jeweiligen Alkalimetallhydroxid umgesetzt. Die Brüden umfassend Alkohol und Wasser werden in mindestens zwei nacheinander geschalteten Rektifikationskolonnen aufgetrennt. Die Energie des in der ersten Rektifikation erhaltenen Brüden wird dabei zum Betrieb der zweiten Rektifikation genutzt. Diese spezifische Energieintegration bei gleichzeitiger Einstellung eines bestimmten Druckunterschieds in den beiden Rektifikationsstufen ermöglicht es, einen besonders großen Anteil der für die Rektifikation benötigten Energie durch Strom zu decken und Heizdampf einzusparen.

### 1. Hintergrund der Erfindung

Die Herstellung von Alkalimetallalkoholaten ist ein wichtiger industrieller Prozess.

Alkalimetallalkoholate werden als starke Basen in der Synthese zahlreicher Chemikalien, z.B. bei der Herstellung von Pharma- oder Agrowirkstoffen, eingesetzt. Weiterhin finden Alkalimetallalkoholate Anwendung als Katalysatoren in Umesterungs- und Amidierungsreaktionen.

Alkalimetallalkoholate (MOR) werden mittels Reaktivdestillation in einer Gegenstromdestillationskolonne aus Alkalimetallhydroxiden (MOH) und Alkoholen (ROH) hergestellt, wobei das gemäß folgender Reaktion <1> entstehende Reaktionswasser mit dem Destillat entfernt wird. MOH + ROH 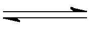 MOR + H₂O.

Ein solches Verfahrensprinzip, nach welchem wässrige Alkalimetallhydroxid-Lösung und gasförmiges Methanol im Gegenstrom in einer Rektifikationskolonne gefahren werden, ist beispielsweise in der US 2,877,274 A offenbart. In prinzipiell nicht veränderter Form wird dieses Verfahren in der WO 01/42178 A1 erneut beschrieben.

Ähnliche Verfahren, bei denen zusätzlich noch ein Schleppmittel, wie z.B. Benzol, eingesetzt wird, sind in der GB 377,631 A und der US 1,910,331 A offenbart. Das Schleppmittel dient hierbei zur Trennung von Wasser und dem wasserlöslichen Alkohol. Bei beiden Patentschriften wird das Kondensat einer Phasentrennung unterzogen, um das Reaktionswasser abzutrennen.

Entsprechend beschreibt die DE 96 89 03 C ein Verfahren zur kontinuierlichen Herstellung von Alkalimetallalkoholaten in einer Reaktionskolonne, wobei das am Kopf entnommene Wasser-Alkohol-Gemisch kondensiert und anschließend einer Phasentrennung unterworfen wird. Die wässrige Phase wird hierbei verworfen, und die alkoholische Phase wird zusammen mit dem frischen Alkohol der Kolonne am Kopf zurückgegeben. Ein ähnliches Verfahren beschreibt die EP 0 299 577 A2, wobei die Wasserabtrennung im Kondensat mit Hilfe einer Membran erfolgt.

Die industriell wichtigsten Alkalimetallalkoholate sind jene des Natriums und Kaliums, und hierbei insbesondere die Methylate und Ethylate. Deren Synthese ist vielfach im Stand der Technik beschrieben, zum Beispiel in der EP 1 997 794 A1.

Bei den im Stand der Technik beschriebenen Synthesen der Alkalimetallalkoholate durch Reaktivrektifikation werden üblicherweise Brüden erhalten, welche den eingesetzten Alkohol und Wasser umfassen. Es ist aus wirtschaftlichen Gründen sinnvoll, den von den Brüden umfassten Alkohol wieder in der Reaktivdestillation als Edukt einzusetzen. Deshalb werden die Brüden üblicherweise einer Rektifikationskolonne zugeführt und der darin enthaltene Alkohol abgetrennt (beschrieben beispielsweise in der GB 737 453 A und der US 4,566,947 A). Der so zurückgewonnene Alkohol wird dann beispielsweise als Edukt der Reaktivdestillation zugeführt. Alternativ oder zusätzlich kann ein Teil des Alkoholbrüden zur Beheizung der Rektifikationskolonne genutzt werden (beschrieben in WO 2010/097318 A1). Dazu muss der Brüden allerdings verdichtet werden, um das zur Beheizung der Rektifikationskolonne nötige Temperaturniveau zu erreichen. Hierbei wird der Brüden zwischen den Verdichtungsstufen gekühlt, wobei eine mehrstufige Verdichtung thermodynamisch vorteilhaft ist und eine Zwischenkühlung dazu beiträgt, die maximal zulässige Temperatur des Verdichters nicht zu überschreiten.

Eine Wärmeintegration innerhalb der Rektifikationsstufe zur effizienten Nutzung eingesetzter Energie beschreiben Ott, J., Gronemann, V., Pontzen, F., Fiedler, E., Grossmann, G., Kersebohm, D.B., Weiss, G. and Witte, C. (2012). Methanol. In Ullmann's Encyclopedia of Industrial Chemistry, (Ed.). (doi:10.1002/14356007.a16_465.pub3) in anderem Zusammenhang. Abschnitt 5.4 dieser Literaturstelle offenbart die Aufarbeitung von in herkömmlichen Syntheseverfahren erhaltenem Rohmethanol durch Rektifikation mittels mehrerer Rektifikationssäulen. Dabei wird allgemein die Nutzung der Kondensationswärme des Brüden, der an der Rektifikationssäule mit höherem Druck erhalten wird, zur Beheizung der Rektifikationssäule mit niedrigerem Druck vorgeschlagen. Zur vorteilhaften Energieintegration bei der Auftrennung von Wasser-Methanol-Brüden, die bei der Reaktivrektifikation von Alkalimetallakoholaten hergestellt wurden, offenbart diese Literaturstelle jedoch nichts.

CN 109 627 145 A beschreibt eine energiesparende Herstellung von Natriummethanolat aus Methanol und NaOH in einer Reaktionskolonne (1) mit Druck p(1). Aus dem Kopfstrom der Reaktionskolonne wird in einer ersten, bei Normaldruck p(2) betriebenen Reaktionskolonne (2) und einer zweiten, bei erhöhtem Druck p(3) betriebenen Reaktionskolonne (3) Methanol abgetrennt und in die Reaktionskolonne (1) zurückgeführt. Das Druckregime ist somit p(3) > p(1) > p(2).

Bei der Herstellung von Alkalimetallalkoholaten ist es im industriellen Maßstab, gerade in Anlagenverbunden (Chemieparks, Technologieparks), möglich, Heizdampf als Energiequelle zur Deckung des Energiebedarfs zu nutzen. Dieser fällt typischerweise bei Anlageverbunden im Überschuss an und kann dann genutzt werden.

Je nach Infrastruktur und verfügbaren Energiequellen ist Heizdampf jedoch nicht immer verfügbar, und in bestimmten Situationen ist Strom günstiger. In diesen Fällen besteht der Bedarf nach Verfahren für die Herstellung von Alkalimetallalkoholaten, bei dem ein möglichst geringer Anteil der Energie durch Heizdampf abgedeckt werden muss und ein möglichst hoher Anteil durch Strom abgedeckt werden kann.

Die Aufgabe der vorliegenden Erfindung bestand demnach darin, ein verbessertes Verfahren zur Herstellung von Alkoholaten des Natriums und Kaliums durch Reaktivdestillation zur Verfügung zu stellen. Dieses sollte insbesondere eine energieeffiziente Nutzung der bei der Verdichtung und Abkühlung der Brüden anfallenden Wärme ermöglichen. Daneben sollte es einen möglichst großen Anteil des Energiebedarfs durch Strom als externe Energiequelle decken und sich durch einen möglichst geringen Heizdampfbedarf auszeichnen.

### 2. Kurzzusammenfassung der Erfindung

Die vorliegende Erfindung betrifft demnach ein Verfahren zur Herstellung mindestens eines Alkalimetallalkoholats der Formel M_{A}OR, wobei R ein C₁ bis C₆-Kohlenwasserstoffrest, bevorzugt Methyl oder Ethyl, ist, und wobei M_{A} ausgewählt aus Natrium, Kalium ist, und wobei M_{A} bevorzugt Natrium ist, wobei:
(a1) ein Eduktstrom **S_{AE1}** umfassend ROH mit einem Eduktstrom **S_{AE2}** umfassend M_{A}OH im Gegenstrom bei einem Druck **p_{3A}** und einer Temperatur **T_{3A}** in einer Reaktivrektifikationskolonne **RR_{A}** zu einem Rohprodukt **RP_{A}** umfassend M_{A}OR, Wasser, ROH, M_{A}OH umgesetzt wird,
wobei am unteren Ende von **RR_{A}** ein Sumpfproduktstrom **S_{AP}** umfassend ROH und M_{A}OR entnommen wird und am oberen Ende von **RR_{A}** ein Brüdenstrom **S_{AB}** umfassend Wasser und ROH entnommen wird,
(a2) und gegebenenfalls, gleichzeitig mit und räumlich getrennt von Schritt (a1), ein Eduktstrom **S_{BE1}** umfassend ROH mit einem Eduktstrom **S_{BE2}** umfassend M_{B}OH im Gegenstrom bei einem Druck **p_{3B}** und einer Temperatur **T_{3B}** in einer Reaktivrektifikationskolonne **RR_{B}** zu einem Rohprodukt **RP_{B}** umfassend M_{B}OR, Wasser, ROH, M_{B}OH umgesetzt wird, wobei M_{B} ausgewählt aus Natrium, Kalium ist, und wobei M_{B} bevorzugt Kalium ist,
wobei am unteren Ende von **RR_{B}** ein Sumpfproduktstrom **S_{BP}** umfassend ROH und M_{B}OR entnommen wird und am oberen Ende von **RR_{B}** ein Brüdenstrom **S_{BB}** umfassend Wasser und ROH entnommen wird,
(b) der Brüdenstrom **S_{AB},** und, wenn Schritt (a2) durchgeführt wird, der Brüdenstrom **S_{BB},** vermischt mit **S_{AB}** oder getrennt von **S_{AB},** in eine erste Rektifikationskolonne **RD₁** geleitet wird,
wodurch ein Gemisch **G_{RD1}** umfassend Wasser und ROH in der ersten Rektifikationskolonne **RD₁** erhalten wird,
(c) das Gemisch **G_{RD1}** in der ersten Rektifikationskolonne **RD₁** bei einem Druck **p₁** und einer Temperatur **T₁** in einen ROH umfassenden Brüdenstrom **S_{RDB1}** am oberen Ende von **RD₁** und einen Sumpfstrom **S_{RDS1}** umfassend Wasser und ROH am unteren Ende von **RD₁** aufgetrennt wird,
(d) der Sumpfstrom **S_{RDS1}** ganz oder teilweise in eine zweite Rektifikationskolonne **RD₂** geleitet wird,
wodurch ein Gemisch **G_{RD2}** umfassend Wasser und ROH in der zweiten Rektifikationskolonne **RD₂** erhalten wird,
(e) das Gemisch **G_{RD2}** bei einem Druck **p₂** und einer Temperatur **T₂** in einen ROH umfassenden Brüdenstrom **S_{RDB2}** am Kopf von **RD₂** und einen Sumpfstrom **S_{RDS2}** umfassend Wasser und gegebenenfalls ROH am unteren Ende von **RD₂** aufgetrennt wird,
dadurch gekennzeichnet,
dass **p₁** > **p₂, p₁** > **p_{3A}** gilt, und in den Fällen, in denen Schritt (a2) durchgeführt wird, **p₁** > **p_{3B}** gilt, und wobei bevorzugt außerdem **p_{3A} > p₂** gilt, und in den Fällen, in denen Schritt (a2) durchgeführt wird, zusätzlich bevorzugt außerdem **p_{3B}** > **p₂** gilt
und dass (f) Energie von **S_{RDB1}** auf das Gemisch **G_{RD2}** in der zweiten Rektifikationskolonne **RD₂** übertragen wird.

### 3. Abbildungen

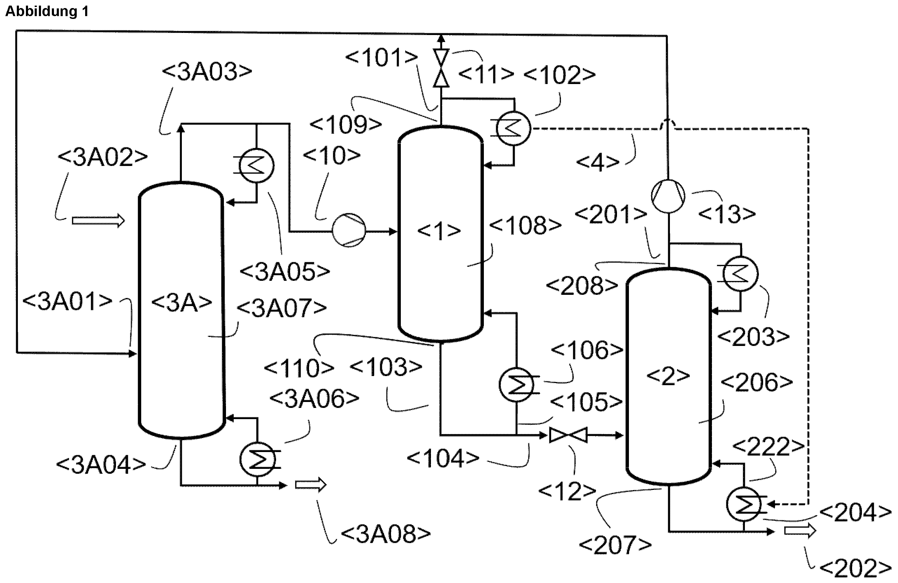

### 3.1 Abbildung 1

Abbildung 1 zeigt ein erfindungsgemäßes Verfahren zur Herstellung von Alkalimetallalkoholaten mit einer entsprechenden Verschaltung der Rektifikationskolonnen. Es werden eine Reaktivrektifikationskolonne ("Reaktivrektifikationskolonne" wird im Folgenden abgekürzt als "Reaktionskolonne") **RR_{A}** <3A> mit Druck **p_{3A}** und zwei Rektifikationskolonnen **RD₁** <1> und **RD₂** <2> mit den Drücken **p₁** bzw. **p₂** eingesetzt. Dabei gilt **p₁** > **p_{3A}** > **p₂.**

In **RR_{A}** <3A> wird NaOH (Strom **S_{AE2}** <3A02>) mit Methanol (Strom **S_{AE1}** <3A01>) zu einem Rohprodukt **RP_{A}** <3A07> umfassend Wasser, Methanol, NaOH und Natriummethanolat umgesetzt. Am unteren Ende von **RR_{A}** <3A> wird ein Methanol-Natriummethanolat-Gemisch **S_{AP}** <3A04> entnommen. Mit dem Sumpfverdampfer **VS_{3A}** <3A06> am unteren Ende der Reaktionskolonne **RR_{A}** <3A> wird die Konzentration der Methanolatlösung auf den gewünschten Wert im dadurch resultierenden Gemisch **S_{AP*}** <3A08> eingestellt. Daneben kann am Sumpf der Reaktionskolonne **RR_{A}** <3A> ein weiterer Verdampfer, insbesondere zum Anfahren der Reaktionskolonne **RR_{A}** <3A>, angebracht sein (nicht gezeigt).

Am Kopf von **RR_{A}** <3A> wird ein Methanol-Wasser-Gemisch als Brüdenstrom **S_{AB}** <3A03> entnommen. **S_{AB}** <3A03> wird der ersten Wasser/Methanol-Kolonne **RD₁** <1> zugeführt, wobei gegebenenfalls **S_{AB}** <3A03> am Kopf der Reaktionskolonne **RR_{A}** <3A> teilweise im Kondensator **K_{RRA}** <3A05> kondensiert und flüssig als Rücklauf auf den Kopf von **RR_{A}** <3A> rückgeführt wird. Zumindest ein Teil des Brüden **S_{AB}** <3A03> wird dann über einen Verdichter **VD₃₁** <10> geleitet, wodurch sich der Druck des Brüden **S_{AB}** <3A03> von **p_{3A}** auf den Druck **p₁** erhöht.

In der ersten Rektifikationskolonne **RD₁** <1> wird dadurch ein Methanol/Wasser-Gemisch **G_{RD1}** <108> erhalten. Methanol wird als Brüden **S_{RDB1}** <101> in dieser ersten Wasser/Methanol-Kolonne **RD₁** <1> destillativ zurückgewonnen. Das als Brüdenstrom **S_{RDB1}** <101> zurückgewonnene Methanol wird an der Entnahmestelle <109> am Kopf von **RD₁** <1> aus dieser geleitet und teilweise am Kopf der Rektifikationskolonne **RD₁** <1> im Kondensator **K_{RD1}** <102> kondensiert und flüssig als Rücklauf auf den Kopf von **RD₁** <1> rückgeführt. Der übrige Teil des als Brüden **S_{RDB1}** <101> zurückgewonnenen Methanols wird zum Beispiel durch eine Drossel **D₁₃** <11> auf den Druck **p₃** entspannt und in **RR_{A}** <3A> als Methanolstrom **S_{AE1}** <3A01> eingespeist.

Am unteren Ende (ein anderer Begriff für "unteres Ende einer Rektifikationskolonne" ist "Sumpf einer Rektifikationskolonne") von **RD₁** <1> wird ein Sumpfstrom **S_{RDS1}** <103> umfassend Wasser und Methanol an der Entnahmestelle <110> abgeleitet. Ein erster Teil **S_{RDS11}** <104> des Stromes **S_{RDS1}** <103> wird einer zweiten Wasser/Methanol-Kolonne **RD₂** <2> zugeführt, ein zweiter Teil **S_{RDS12}** <105> des Stromes **S_{RDS1}** <103> wird über einen Sumpfverdampfer **VS_{RD1}** <106> in **RD₁** <1> zurückgeführt. **S_{RDS11}** <104> wird z.B. durch eine Drossel **D₁₂** <12> auf den Druck **p₂** entspannt, bevor er in **RD₂** <2> eingespeist wird.

In der zweiten Rektifikationskolonne **RD₂** <2> wird dadurch ein Methanol/Wasser-Gemisch **G_{RD2}** <206> erhalten. In der Rektifikationskolonne **RD₂** <2> werden Rückstände von Methanol aus **S_{RDS11}** <104> vom Wasser abgetrennt und als Brüdenstrom **S_{RDB2}** <201> am Kopf von **RD₂** <2> destillativ zurückgewonnen. Das als Brüdenstrom **S_{RDB2}** <201> zurückgewonnene Methanol wird an der Entnahmestelle <208> am Kopf von **RD₂** <2> aus dieser geleitet und teilweise am Kopf der Rektifikationskolonne **RD₂** <2> im Kondensator **K_{RD2}** <203> kondensiert und flüssig als Rücklauf auf den Kopf von **RD₂** <2> zurückgeführt. Der übrige Teil des als Brüden **S_{RDB2}** <201> zurückgewonnenen Methanols wird über einen Verdichter **VD₂₃** <13> geleitet, dadurch auf den Druck **p₃** verdichtet und zusammen mit dem auf den Druck **p₃** entspannten Brüden **S_{RDB1}** <101> aus **RD₁** <1> als Methanolstrom **S_{AE1}** <3A01> in **RR_{A}** <3A> eingespeist.

Am unteren Ende von **RD₂** <2> wird ein Sumpfstrom **S_{RDS2}** <202> umfassend Wasser und gegebenenfalls Methanol an der Entnahmestelle <207> abgeleitet. Ein Teil **S_{RDS22}** <222> von **S_{RDS2}** <202> wird über einen Sumpfverdampfer **VS_{RD2}** <204> beheizt und in **RD₂** <2> zurückgeführt.

Für die Beheizung des Teils des Sumpfstrom **S_{RDS2}** <202>, der über **VS_{RD2}** <204> in **RD₂** <2> rückgeführt wird, wird dabei die bei der Kondensation von **S_{RDB1}** <101> im Kondensator **K_{RD1}** <102> am Kopf der Rektifikationskolonne **RD₁** <1> anfallende Energie genutzt. Diese wird **VS_{RD2}** <204> zugeführt, wie durch den gestrichelten Pfeil <4> angedeutet. Dabei kann die Zuführung indirekt, d.h. unter Zuhilfenahme eines von **S_{RDB1}** <101> und **S_{RDS2}** <202> verschiedenen Wärmeträgers, oder auch direkt, das heißt durch Kontaktierung von **S_{RDB1}** <101> mit **S_{RDS2}** <202> im Kondensator **K_{RD1}** <102> oder Sumpfverdampfer **VS_{RD2}** <204>, erfolgen. Bei der direkten Kontaktierung reicht es aus, nur den Kondensator **K_{RD1}** <102> einzusetzen und den Sumpfverdampfer **VS_{RD2}** <204> wegzulassen oder nur den Sumpfverdampfer **VS_{RD2}** <204> einzusetzen und den Kondensator **K_{RD1}** <102> wegzulassen, und beide Ströme **S_{RDB1}** <101> mit **S_{RDS2}** <202> dann jeweils so durch den Kondensator **K_{RD1}** <102> oder den Sumpfverdampfer **VS_{RD2}** <204> zu führen, dass Energie, bevorzugt Wärme, von **S_{RDB1}** <101> auf **S_{RDS2}** <202> übertragen wird.

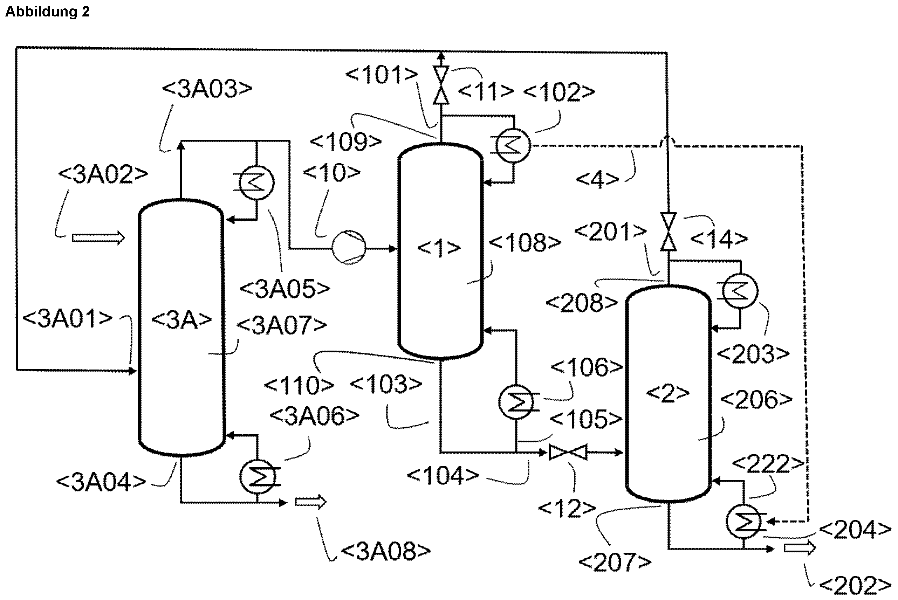

### 3.2 Abbildung 2

Abbildung 2 zeigt ein weiteres erfindungsgemäßes Verfahren zur Herstellung von Alkalimetallalkoholaten. Dieses unterscheidet sich von dem in Abbildung 1 dargestellten Verfahren in den in der jeweiligen Kolonne vorliegenden Drücken. In der in Abbildung 1 dargestellten Ausführungsform gilt **p₁** > **p_{3A}** > **p₂,** wohingegen in der in Abbildung 2 dargestellten Ausführungsform **p₁** > **p₂** > **p_{3A}** gilt. Durch dieses unterschiedliche Druckregime ist der Verdichter **VD₂₃** <13> unnötig, und es wird z.B. eine Drossel **D₂₃** <14> angebracht. Durch die Drossel **D₂₃** <14> wird der Brüdenstrom **S_{RDB2}** <201> von **p₂** auf den Druck **p_{3A}** entspannt, während er in der Ausführungsform gemäß Abbildung 1 durch den Verdichter **VD₂₃** <13> von **p₂** auf den Druck **p_{3A}** erhöht wird.

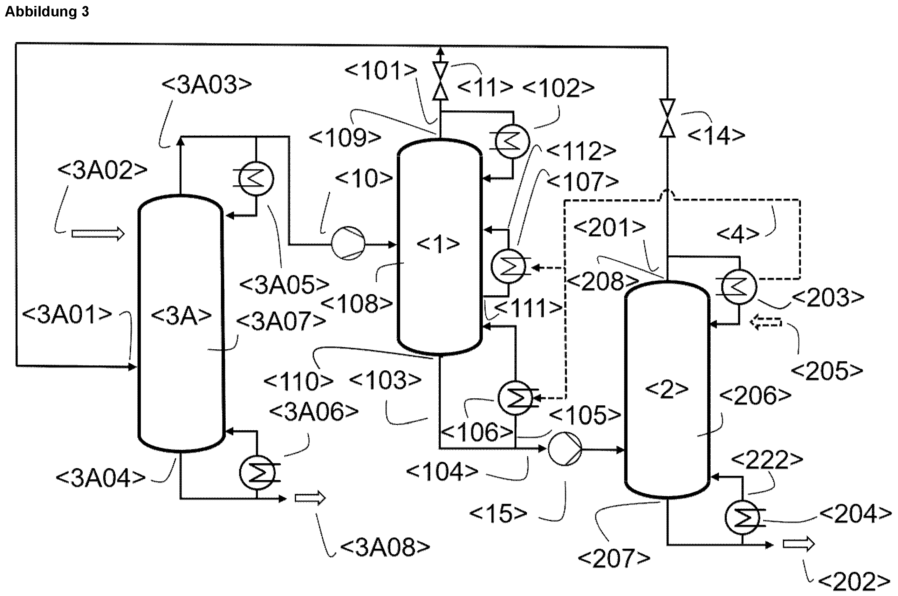

### 3.3 Abbildung 3

Abbildung 3 zeigt eine Ausführungsform eines nicht erfindungsgemäßen Verfahrens zur Herstellung von Alkalimetallalkoholaten mit einer entsprechenden Verschaltung der Reaktivrektifikations- und Rektifikationskolonnen. Dabei werden, wie auch in den in Abbildungen 1 und 2 beschriebenen Ausführungsformen, eine Reaktivrektifikationskolonne **RR_{A}** <3A> mit Druck **p_{3A}** und zwei Rektifikationskolonnen **RD₁** <1> und **RD₂** <2> aufweisend die Drücke **p₁** bzw. **p₂** eingesetzt. Hier gilt **p₂** > **p₁** > **p_{3A}.** Der in Abbildung 3 gezeigte Aufbau entspricht dem in Abbildung 2 gezeigten Aufbau mit folgenden Unterschieden:
1. An der Rektifikationskolonne **RD₁** <1> befindet sich neben dem Sumpfverdampfer **VS_{RD1}** <106> ein Zwischenverdampfer **VZ_{RD1}** <107>, mit welchem dem Gemisch **G_{RD1}** <108> in **RD₁** <1> Energie zugeführt werden kann. Dazu wird das Gemisch **G_{RD1}** <108> an einer Entnahmestelle <111> der Rektifikationskolonne **RD₁** <1> als Strom **S_{RDX1}** <112> abgeleitet. **S_{RDX1}** <112> wird in **VZ_{RD1}** <107> erhitzt und in die Rektifikationskolonne **RD₁** <1> zurückgeleitet. Ein entsprechender Zwischenverdampfer kann auch an **RD₂** <2> in den Ausführungsformen gemäß Beispielen 1 und 2 angebracht sein.
2. Die Drossel **D₁₂** <12> wird aufgrund der unterschiedlichen Drücke in den Rektifikationskolonnen **RD₁** <1> und **RD₂** <2> (**p₂** > **p₁**) durch eine Pumpe **P** <15> ersetzt. Der Grund für diesen Unterschied ist, dass der Druck von **S_{RDS11}** <104>, wenn dieser Strom in **RD₂** <2> geleitet wird, erfindungsgemäß auf **p₂** erhöht wird.
3. Als optionale Ausführungsform wird zusätzliches Methanol als Strom **S_{XE1}** <205> über den Rücklauf an der Rektifikationskolonne **RD₂** <2> ebendieser zugegeben.
4. Die Energie, die bei der Kondensation des Brüden **S_{RDB2}** <201> am Kopf von **RD₂** <2> anfällt, wird über den Zwischenverdampfer **VZ_{RD1}** <107> auf **S_{RDX1}** <112> übertragen und, nachdem **S_{RDX1}** <112> wieder in **RD₁** <1> eingespeist wird, von **S_{RDX1}** <112> auf das in **RD₁** <1> befindliche Gemisch **G_{RD1}** <108> übertragen. Alternativ oder zusätzlich wird Energie, die bei der Kondensation des Brüden **S_{RDB2}** <201> am Kopf von **RD₂** <2> anfällt, über den Sumpfverdampfer **VS_{RD1}** <106> auf den Teil **S_{RDS12}** <105> des Stromes **S_{RDS1}** <103> übertragen. Nachdem **S_{RDS12}** <105> in **RD₁** <1> zurückgeführt wird, überträgt es die Energie auf das in **RD₁** <1> befindliche Gemisch **G_{RD1}** <108>. Der Energiefluss ist durch den gestrichelten Pfeil <4> gezeigt.

Bei der direkten Kontaktierung reicht es aus, nur den Kondensator **K_{RD2}** <203> einzusetzen und den Sumpfverdampfer **VS_{RD1}** <106> wegzulassen oder nur den Sumpfverdampfer **VS_{RD1}** <106> einzusetzen und den Kondensator **K_{RD2}** <203> wegzulassen, und beide Ströme **S_{RDB2}** <201> mit **S_{RDS12}** <105> dann jeweils so durch den Kondensator **K_{RD2}** <203> oder den Sumpfverdampfer **VS_{RD1}** <106> zu führen, dass Energie, bevorzugt Wärme, von **S_{RDB2}** <201> auf **S_{RDS12}** <105> übertragen wird.

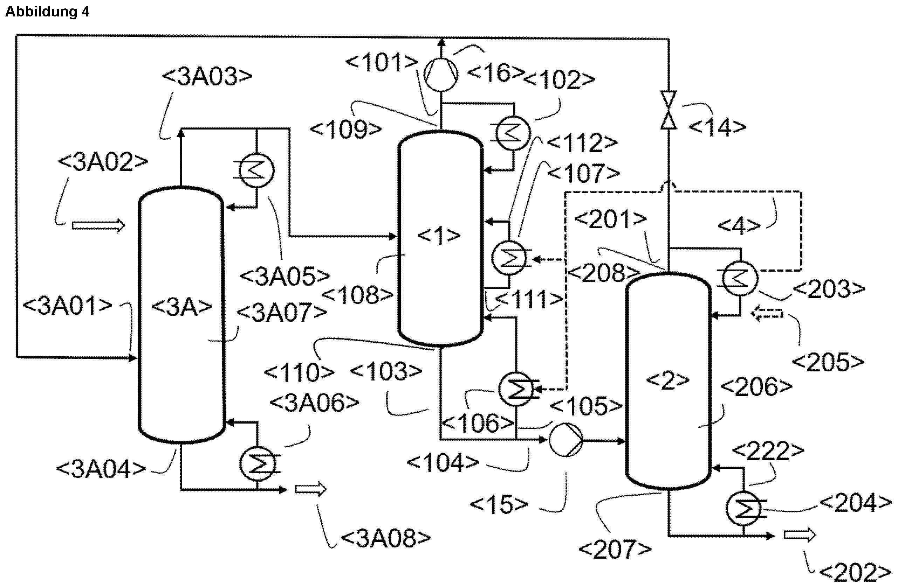

### 3.4 Abbildung 4

Abbildung 4 zeigt eine weitere Ausführungsform eines nicht erfindungsgemäßen Verfahrens zur Herstellung von Alkalimetallalkoholaten mit einer entsprechenden Verschaltung der Rektifikationskolonnen. Dabei werden, wie auch in den in Abbildungen 1 und 2 beschriebenen Ausführungsformen, eine Reaktivrektifikationskolonne **RR_{A}** <3A> mit Druck **p_{3A}** und zwei Rektifikationskolonnen **RD₁** <1> und **RD₂** <2> aufweisend die Drücke **p₁** bzw. **p₂** eingesetzt. Hier gilt **p₂** > **p_{3A}** > **p₁.** Der in Abbildung 4 gezeigte Aufbau entspricht dem in Abbildung 3 gezeigten Aufbau bis auf den Unterschied, dass der Druck **p_{3A}** > **p₁.** Dadurch kann der Verdichter **VD₃₁** <10> weggelassen werden, während die Drossel **D₁₃** <11> durch den Verdichter **VD₁₃** <16> ersetzt wird.

### 4. Detaillierte Beschreibung der Erfindung

### 4.1 Schritt (a1) des erfindungsgemäßen Verfahrens

Im Schritt (a1) des erfindungsgemäßen Verfahrens zur Herstellung mindestens eines Alkalimetallalkoholats der Formel M_{A}OR wird ein Eduktstrom **S_{AE1}** umfassend ROH mit einem Eduktstrom **S_{AE2}** umfassend M_{A}OH im Gegenstrom bei einem Druck **p_{3A}** und einer Temperatur **T_{3A}** in einer Reaktivrektifikationskolonne **RR_{A}** zu einem Rohprodukt **RP_{A}** umfassend M_{A}OR, Wasser, ROH, M_{A}OH umgesetzt.

Als "Reaktivrektifikationskolonne" wird erfindungsgemäß eine Rektifikationskolonne definiert, in der zumindest in einigen Teilen die Umsetzung nach Schritt (a1) oder Schritt (a2) des erfindungsgemäßen Verfahrens ablaufen. Sie kann auch als "Reaktionskolonne" abgekürzt werden.

In Schritt (a1) des erfindungsgemäßen Verfahrens wird am unteren Ende von **RR_{A}** ein Sumpfproduktstrom **S_{AP}** umfassend ROH und M_{A}OR entnommen. Am oberen Ende von **RR_{A}** wird ein Brüdenstrom **S_{AB}** umfassend Wasser und ROH entnommen.

"Brüdenstrom" bedeutet, dass es sich bei dem jeweiligen Strom um einen gasförmigen Strom handelt.

R ist im erfindungsgemäßen Verfahren ein C₁ bis C₆-Kohlenwasserstoffrest, bevorzugt ausgewählt aus der Gruppe bestehend aus Methyl, Ethyl, *n*-Propyl, *iso*-Propyl, *n*-Butyl, *sec*-Butyl, *iso*-Butyl, *tert*-Butyl, Isomeren des Pentyls, wie beispielsweise *n*-Pentyl, bevorzugter ausgewählt aus der Gruppe bestehend aus Methyl, Ethyl, *n*-Propyl, *iso*-Propyl, noch bevorzugter ausgewählt aus der Gruppe bestehend aus Methyl, Ethyl. Besonders bevorzugt ist R Methyl und ROH demnach Methanol.

M_{A} ist ausgewählt aus Natrium, Kalium, und bevorzugt Natrium.

Der Eduktstrom **S_{AE1}** umfasst ROH. In einer bevorzugten Ausführungsform liegt der Massenanteil von ROH in **S_{AE1},** bezogen auf die gesamte Masse des Eduktstroms **S_{AE1},** bei ≥ 95 Gew.-%, noch bevorzugter bei ≥ 99 Gew.-%, wobei insbesondere **S_{AE1}** ansonsten Wasser aufweist.

Der in Schritt (a1) des erfindungsgemäßen Verfahrens als Eduktstrom **S_{AE1}** eingesetzte Alkohol ROH kann auch handelsüblicher Alkohol mit einem Alkoholmassenanteil, bezogen auf die gesamte Masse des Eduktstroms **S_{AE1},** von mehr als 99.8 Gew.-% und einem Massenanteil an Wasser, bezogen auf die gesamte Masse des Eduktstroms **S_{AE1},** von bis zu 0.2 Gew.-% sein.

Der Eduktstrom **S_{AE1}** wird bevorzugt dampfförmig zugegeben.

Der Eduktstrom **S_{AE2}** umfasst M_{A}OH. In einer bevorzugten Ausführungsform umfasst **S_{AE2}** neben M_{A}OH mindestens eine weitere Verbindung ausgewählt aus Wasser, ROH. Noch bevorzugter umfasst **S_{AE2}** neben M_{A}OH auch Wasser, dann handelt es sich bei **S_{AE2}** um eine wässrige Lösung von M_{A}OH.

Wenn der Eduktstrom **S_{AE2}** M_{A}OH und Wasser umfasst, liegt der Massenanteil von M_{A}OH, bezogen auf das Gesamtgewicht des Eduktstroms **S_{AE2},** insbesondere im Bereich von 10 bis 55 Gew.-%, bevorzugt von 15 bis 54 Gew.-%, bevorzugter von 30 bis 53 Gew.-% und besonders bevorzugt von 45 bis 52 Gew.-%, am bevorzugtesten bei 50 Gew.-%.

Wenn der Eduktstrom **S_{AE2}** M_{A}OH und ROH umfasst, liegt der Massenanteil von M_{A}OH, bezogen auf das Gesamtgewicht des Eduktstroms **S_{AE2},** insbesondere im Bereich von 10 bis 55 Gew.-%, bevorzugt von 15 bis 54 Gew.-%, bevorzugter von 30 bis 53 Gew.-%, und besonders bevorzugt von 45 bis 52 Gew.-%.

In dem besonderen Fall, in dem der Eduktstrom **S_{AE2}** neben M_{A}OH sowohl Wasser als auch ROH umfasst, ist es besonders bevorzugt, dass der Massenanteil von M_{A}OH, bezogen auf das Gesamtgewicht des Eduktstroms **S_{AE2},** insbesondere im Bereich von 10 bis 55 Gew.-%, bevorzugt von 15 bis 54 Gew.-%, bevorzugter von 30 bis 53 Gew.-%, und besonders bevorzugt von 45 bis 52 Gew.-% liegt.

Schritt (a1) des erfindungsgemäßen Verfahrens wird in einer Reaktivrektifikationskolonne (oder "Reaktionskolonne") **RR_{A}** durchgeführt.

Schritt (a2) des erfindungsgemäßen Verfahrens wird in einer Reaktivrektifikationskolonne (oder "Reaktionskolonne") **RR_{B}** durchgeführt.

Bevorzugt enthält die Reaktionskolonne **RR_{A}** bzw. **RR_{B}** Einbauten. Geeignete Einbauten sind zum Beispiel Böden, strukturierte Packungen oder unstrukturierte Packungen. Wenn die Reaktionskolonne **RR_{A}** bzw. **RR_{B}** Böden enthält, so sind Glockenböden, Ventilböden, Tunnelböden, Thormann-Böden, Kreuzschlitzglockenböden oder Siebböden geeignet. Wenn die Reaktionskolonne **RR_{A}** bzw. **RR_{B}** Böden enthält, so werden vorzugsweise solche Böden gewählt, bei denen maximal 5 Gew.-%, bevorzugt weniger als 1 Gew.-% der Flüssigkeit durch die jeweiligen Böden durchregnen. Die zur Minimierung des Durchregnens der Flüssigkeit erforderlichen konstruktiven Maßnahmen sind dem Fachmann geläufig. Bei Ventilböden werden zum Beispiel besonders dicht schließende Ventilbauarten gewählt. Durch Verringerung der Zahl der Ventile lässt sich zudem die Dampfgeschwindigkeit in den Bodenöffnungen auf das Doppelte des Wertes, der üblicherweise eingestellt wird, erhöhen. Bei Einsatz von Siebböden ist es besonders günstig, die Durchmesser der Bodenöffnungen zu verringern und die Zahl der Öffnungen beizubehalten oder noch zu vergrößern.

Bei Einsatz von strukturierten oder unstrukturierten Packungen sind strukturierte Packungen im Hinblick auf die gleichmäßige Verteilung der Flüssigkeit bevorzugt. Bei dieser Ausführungsform ist es zudem bevorzugt, dass in allen Teilen des Kolonnenquerschnitts, die mehr als 2 % des gesamten Kolonnenquerschnitts entsprechen, das gemittelte Verhältnis von Flüssigkeits- zu Dampfstrom hinsichtlich der Flüssigkeit um nicht mehr als 15 %, bevorzugter um nicht mehr als 3 % überschritten werden. Diese niedrig gehaltene Flüssigkeitsmenge macht es möglich, dass der Kapillareffekt an den Drahtgeweben lokale Spitzenwerte der Flüssigkeitsberieselungsdichte ausschließt.

Bei Kolonnen mit unstrukturierten Packungen, insbesondere mit Füllkörpern, und bei Kolonnen mit strukturierten Packungen kann die gewünschte Charakteristik der Flüssigkeitsverteilung dadurch erzielt werden, dass in dem dem Kolonnenmantel benachbarten Randbereich des Kolonnenquerschnitts, der etwa 2 bis 5 % des gesamten Kolonnenquerschnitts entspricht, die Flüssigkeitsberieselungsdichte gegenüber den übrigen Querschnittsbereichen um bis zu 100 %, bevorzugt um 5 bis 15 %, verringert wird. Dies lässt sich zum Beispiel durch die gezielte Verteilung der Abtropfstellen der Flüssigkeitsverteiler oder deren Bohrungen mit einfachen Mitteln erreichen.

Das erfindungsgemäße Verfahren kann sowohl kontinuierlich als auch diskontinuierlich erfolgen. Bevorzugt erfolgt es kontinuierlich.

"Umsetzung eines Eduktstroms **S_{AE1}** umfassend ROH mit einem Eduktstrom **S_{AE2}** umfassend M_{A}OH im Gegenstrom" wird erfindungsgemäß insbesondere dadurch gewährleistet, dass der Zugabepunkt mindestens eines Teils des Eduktstroms **S_{AE1}** umfassend ROH in Schritt (a1) an der Reaktionskolonne **RR_{A}** unterhalb des Zugabepunktes des Eduktstroms **S_{AE2}** umfassend M_{A}OH liegt.

Die Reaktionskolonne **RR_{A}** umfasst vorzugsweise mindestens 2, insbesondere 15 bis 40 theoretische Böden zwischen dem Zugabepunkt des Eduktstroms **S_{AE1}** und dem Zugabepunkt des Eduktstroms **S_{AE2}.**

Die Reaktionskolonne **RR_{A}** wird bevorzugt als reine Abtriebskolonne betrieben. Insbesondere wird demnach im unteren Bereich der Reaktionskolonne **RR_{A}** dampfförmig der Eduktstrom **S_{AE1}** umfassend ROH zugeführt. Schritt (a1) des erfindungsgemäßen Verfahrens umfasst auch den Fall, dass ein Teil des Eduktstroms **S_{AE1}** umfassend ROH zwar unterhalb des Zugabepunktes des Eduktstroms **S_{AE2}** umfassend M_{A}OH, aber dennoch am oberen Ende oder im Bereich des oberen Endes der Reaktionskolonne **RR_{A}** dampfförmig zugegeben wird. Dadurch können die Abmessungen im unteren Bereich der Reaktionskolonne **RR_{A}** verringert werden. Wenn ein Teil des Eduktstroms **S_{AE1}** umfassend ROH, insbesondere Methanol, am oberen Ende oder im Bereich des oberen Endes der Reaktionskolonne **RR_{A}** insbesondere dampfförmig zugegeben wird, so wird nur eine Teilmenge von 10 bis 70 Gew.-%, bevorzugt von 30 bis 50 Gew.-% (jeweils bezogen auf die Gesamtmenge des in Schritt (a1) als **S_{AE1}** eingesetzten Alkohols ROH) am unteren Ende der Reaktionskolonne **RR_{A}** eingespeist und die restliche Teilmenge in einem Einzelstrom oder auf mehrere Teilströme verteilt, bevorzugt 1 bis 10 theoretische Böden, besonders bevorzugt 1 bis 3 theoretische Böden unterhalb des Zugabepunktes des Eduktstroms **S_{AE2}** umfassend M_{A}OH dampfförmig zugegeben.

In der Reaktionskolonne **RR_{A}** setzt sich dann der Eduktstrom **S_{AE1}** umfassend ROH mit dem Eduktstrom **S_{AE2}** umfassend M_{A}OH gemäß der vorstehend beschriebenen Reaktion <1> zu M_{A}OR und H₂O um, wobei, da es sich um eine Gleichgewichtsreaktion handelt, diese Produkte in Mischung mit den Edukten ROH und M_{A}OH vorliegen. Demnach wird in Schritt (a1) des erfindungsgemäßen Verfahrens in der Reaktionskolonne **RR_{A}** ein Rohprodukt **RP_{A}** erhalten, welches neben den Produkten M_{A}OR und Wasser auch noch ROH und M_{A}OH umfasst.

Am unteren Ende von **RR_{A}** erhält und entnimmt man dann den Sumpfproduktstrom **S_{AP}** umfassend ROH und M_{A}OR.

Am oberen Ende von **RR_{A},** bevorzugt am Kolonnenkopf von **RR_{A},** entnimmt man noch Wasser enthaltenden Alkoholstrom, vorstehend als "Brüdenstrom **S_{AB}** umfassend Wasser und ROH" bezeichnet.

Die Menge des vom Eduktstrom **S_{AE1}** umfassten Alkohols ROH wird vorzugsweise so gewählt, dass dieser gleichzeitig als Lösungsmittel für das im Sumpfproduktstrom **S_{AP}** erhaltene Alkalialkoholat M_{A}OR dient. Vorzugsweise wird die Menge des Alkohols ROH im Eduktstrom **S_{AE1}** so gewählt, dass im Sumpf der Reaktionskolonne **RR_{A}** die gewünschte Konzentration der Alkalialkoholat-Lösung vorliegt, die als Sumpfproduktstrom **S_{AP}** umfassend ROH und M_{A}OR entnommen wird.

In einer bevorzugten Ausführungsform des erfindungsgemäßen Verfahrens, und insbesondere in den Fällen, in denen **S_{AE2}** neben M_{A}OH auch Wasser umfasst, beträgt das Verhältnis aus dem Gesamtgewicht (Massen; Einheit: kg) an in Schritt (a1) als Eduktstrom **S_{AE1}** eingesetztem Alkohol ROH zum Gesamtgewicht (Massen; Einheit: kg) an in Schritt (a1) als Eduktstrom **S_{AE2}** eingesetzten M_{A}OH 1 : 1 bis 50 : 1, bevorzugter 5 : 1 bis 48 : 1, noch bevorzugter 9 : 1 bis 35 : 1, noch mehr bevorzugter 10 : 1 bis 30 : 1, noch mehr bevorzugter 13 : 1 bis 22 : 1, am bevorzugtesten 14 : 1.

Die Reaktionskolonne **RR_{A}** wird mit oder ohne, vorzugsweise ohne Rücklauf betrieben.

"Ohne Rücklauf" bedeutet, dass der am oberen Ende von **RR_{A}** entnommene Brüdenstrom **S_{AB}** umfassend Wasser und ROH der ersten Rektifikationskolonne **RD₁** gemäß Schritt (b) vollständig zugeführt wird. Der Brüdenstrom **S_{AB}** umfassend Wasser und ROH wird der ersten Rektifikationskolonne **RD₁** dabei vorzugsweise dampfförmig zugeführt.

"Mit Rücklauf" bedeutet, dass der am oberen Ende der jeweiligen Kolonne, in Schritt (a1) der Reaktionskolonne **RR_{A},** entnommene Brüdenstrom **S_{AB}** umfassend Wasser und ROH nicht vollständig abgeführt wird, in Schritt (b) also nicht vollständig der ersten Rektifikationskolonne **RD₁** zugeführt wird, sondern mindestens teilweise, bevorzugt teilweise, wieder als Rücklauf der jeweiligen Kolonne, in Schritt (a1) also der Reaktionskolonne **RR_{A},** zugeführt wird. In den Fällen, in denen ein solcher Rücklauf eingestellt wird, beträgt das Rücklaufverhältnis dabei bevorzugt 0.05 bis 0.99, bevorzugter 0.1 bis 0.9, noch bevorzugter 0.11 bis 0.34, besonders bevorzugt 0.14 bis 0.27 und ganz besonders bevorzugt 0.17 bis 0.24. Ein Rücklauf kann dadurch eingestellt werden, dass am Kopf der jeweiligen Kolonne, in Schritt (a1) der Reaktionskolonne **RR_{A},** ein Kondensator **K_{RRA}** angebracht wird, in welchem der Brüdenstrom **S_{AB}** mindestens teilweise kondensiert wird und der jeweiligen Kolonne, in Schritt (a1) der Reaktionskolonne **RR_{A},** wieder zugeführt wird. Unter einem Rücklaufverhältnis wird allgemein und im Sinne dieser Erfindung das Verhältnis des Massenstroms (kg/h) verstanden, der der jeweiligen Kolonne in flüssiger Form zurückgeführt wird (Rücklauf) zu dem Massenstrom (kg/h), der in flüssiger Form (Destillat) oder gasförmiger Form (Brüden) von der jeweiligen Kolonne abgeführt wird.

In der Ausführungsform, in der an der Reaktionskolonne **RR_{A}** ein Rücklauf eingestellt wird, kann der in Schritt (a1) als Eduktstrom **S_{AE2}** eingesetzte Alkohol M_{A}OH auch mindestens teilweise, bevorzugt teilweise, mit dem Rücklaufstrom vermischt werden und die resultierende Mischung so dem Schritt (a1) zugeführt werden.

Der Schritt (a1) des erfindungsgemäßen Verfahrens wird insbesondere bei einer Temperatur **T_{3A}** im Bereich von 25 °C bis 200 °C, bevorzugt im Bereich von 45 °C bis 150 °C, bevorzugter im Bereich von 47 °C bis 120 °C, bevorzugter im Bereich von 60 °C bis 110 °C, durchgeführt.

Der Schritt (a1) des erfindungsgemäßen Verfahrens wird insbesondere bei einem Druck **p_{3A}** von 0.5 bar bis 40 bar, bevorzugt im Bereich von 0.75 bar bis 5 bar, bevorzugter im Bereich von 1 bar bis 2 bar, bevorzugter im Bereich von 1 bar bis 1.8 bar, noch bevorzugter bei 1.1 bar bis 1.6 bar durchgeführt. Dabei ist erfindungswesentlich, dass bei der Einstellung des Druckes **p_{3A}** gilt: **p₁** > **p_{3A}.** Insbesondere gilt außerdem **p_{3A}** > **p₂.**

Die Reaktionskolonne **RR_{A}** umfasst in einer bevorzugten Ausführungsform mindestens einen Verdampfer, der insbesondere aus Zwischenverdampfern **VZ_{3A}** und Sumpfverdampfern **VS_{3A}** ausgewählt ist. Die Reaktionskolonne **RR_{A}** umfasst besonders bevorzugt mindestens einen Sumpfverdampfer **VS_{3A}.** Verdampfer sind spezielle Ausführungsformen von Wärmeüberträgern **WT.**

Kondensatoren K sind ebenfalls spezielle Ausführungsformen von Wärmeüberträgern **WT.** Typische Kondensatoren sind dem Fachmann bekannt. Diese werden bevorzugt als Verflüssiger am Kopf von Rektifikationskolonnen und Reaktionskolonnen eingesetzt. Bei der direkten Energieübertragung vom Kopfstrom einer Kolonne auf den Sumpf- oder Zwischenstrom einer anderen Kolonne kann ein Kondensator einer Kolonne gleichzeitig als Verdampfer der anderen Kolonne eingesetzt werden (wie in den Beispielen gezeigt).

Als "Zwischenverdampfer" VZ (z.B. **VZ_{3A}** bei **RR_{A}, VZ_{3B}** bei **RR_{B}, VZ_{RD1}** bei **RD₁, VZ_{RD2}** bei **RD₂**) werden erfindungsgemäß Verdampfer bezeichnet, die sich oberhalb des Sumpfs der jeweiligen Kolonne, insbesondere oberhalb des Sumpfs der Reaktionskolonne **RR_{A}** bzw. **RR_{B},** bzw. oberhalb des Sumpfes der Rektifikationskolonne **RD₁** oder **RD₂** befinden. In ihnen wird insbesondere Rohprodukt **RP_{A}** bzw. **RP_{B}** oder **S_{RDX1Z}** als Seitenstrom verdampft.

Als "Sumpfverdampfer" VS (z.B. **VS_{3A}** bei **RR_{A}, VS_{3B}** bei **RR_{B}, VS_{RD1}** bei **RD₁, VS_{RD2}** bei **RD₂**) werden erfindungsgemäß Verdampfer bezeichnet, die den Sumpf der jeweiligen Kolonne, insbesondere den Sumpf der Reaktionskolonne **RR_{A}** bzw. **RR_{B}** oder Rektifikationskolonne **RD₁** oder **RD₂** beheizen. In ihnen wird Sumpfproduktstrom (z.B. **S_{AP}** bzw. **S_{BP}** oder **S_{RDX1S}**) verdampft.

Ein Verdampfer ist üblicherweise außerhalb der jeweiligen Reaktionskolonne oder Rektifikationskolonne angeordnet. Über einen Abzug oder "Entnahmestelle" aus der Kolonne wird das im Verdampfer zu verdampfende Gemisch abgezogen und dem mindestens einen Verdampfer zugeführt.

Über einen Zulauf oder "Zugabestelle" wird das verdampfte Gemisch, gegebenenfalls mit einem Restanteil Flüssigkeit, wieder in die jeweilige Kolonne zurückgeführt. Wenn der Verdampfer ein Zwischenverdampfer ist, ist der Abzug, über den das jeweilige Gemisch abgezogen und dem Verdampfer zugeführt wird, ein Seitenabzug und der Zulauf, über den das verdampfte jeweilige Gemisch der Kolonne wieder zugeführt wird, ein Seitenzulauf. Wenn der Verdampfer ein Sumpfverdampfer ist, also den Kolonnensumpf beheizt, so wird zumindest ein Teil des Sumpfabzugsstroms verdampft und im Bereich des Sumpfs wieder in die jeweilige Kolonne zurückgeführt.

Alternativ ist es jedoch auch möglich, zum Beispiel auf einem geeigneten Boden bei Einsatz eines Zwischenverdampfers oder im Sumpf der jeweiligen Kolonne Rohre auszubilden, die von dem entsprechenden Wärmemedium durchströmt werden. In diesem Fall erfolgt die Verdampfung auf dem Boden bzw. im Sumpf der Kolonne. Bevorzugt ist es jedoch, den Verdampfer außerhalb der jeweiligen Kolonne anzuordnen.

Geeignete Verdampfer, die als Zwischenverdampfer und Sumpfverdampfer eingesetzt werden können, sind zum Beispiel Naturumlaufverdampfer, Zwangsumlaufverdampfer, Zwangsumlaufverdampfer mit Entspannung, Dampfkessel, Fallfilmverdampfer oder Dünnschichtverdampfer. Als Wärmeübertrager für den Verdampfer wird bei Naturumlaufverdampfern und Zwangsumlaufverdampfern üblicherweise ein Rohrbündel oder Plattenapparat eingesetzt. Bei Einsatz eines Rohrbündelüberträgers kann das Wärmemedium entweder durch die Rohre strömen und das zu verdampfende Gemisch die Rohre umströmen oder aber das Wärmemedium umströmt die Rohre und das zu verdampfende Gemisch durchströmt die Rohre. Bei einem Fallfilmverdampfer wird das zu verdampfende Gemisch üblicherweise als dünner Film auf der Innenseite eines Rohres zugegeben und das Rohr wird von außen beheizt. Im Unterschied zu einem Fallfilmverdampfer ist in einem Dünnschichtverdampfer zusätzlich ein Rotor mit Wischern vorgesehen, der die zu verdampfende Flüssigkeit auf der Innenwand des Rohres zu einem dünnen Film verteilt.

Neben den genannten kann aber auch jede beliebige andere, dem Fachmann bekannte Verdampferbauart, die sich zum Einsatz an einer Rektifikationskolonne eignet, eingesetzt werden.

Wenn die Reaktionskolonne **RR_{A}** bzw. Reaktionskolonne **RR_{B}** einen Zwischenverdampfer **VZ_{3A}** bzw. **VZ_{3B}** aufweist, ist es bevorzugt, wenn der jeweilige Zwischenverdampfer im Abtriebsteil der Reaktionskolonne **RR_{A}** im Bereich der Zulaufstelle des Eduktstroms **S_{AE1}** bzw. im Falle der Reaktionskolonne **RR_{B}** im Bereich der Zulaufstelle des Eduktstroms **S_{BE1}** angeordnet ist. Dadurch kann ein überwiegender Teil der Heizenergie durch den Zwischenverdampfer **VZ_{3A}** bzw. **VZ_{3B}** eingebracht werden. So ist es zum Beispiel möglich, über 80% der Energie über den Zwischenverdampfer einzubringen. Erfindungsgemäß wird der Zwischenverdampfer **VZ_{3A}** bzw. **VZ_{3B}** vorzugsweise so angeordnet und/oder gestaltet, dass mit diesem mehr als 50 %, insbesondere mehr als 75 % der für die Reaktivrektifikation benötigten Gesamtenergie eingebracht werden.

Wenn die Reaktionskolonne **RR_{A}** bzw. Reaktionskolonne **RR_{B}** einen Zwischenverdampfer **VZ_{3A}** bzw. **VZ_{3B}** aufweist, ist es außerdem vorteilhaft, wenn der Zwischenverdampfer so angeordnet ist, dass die Reaktionskolonne **RR_{A}** bzw. **RR_{B}** unterhalb des Zwischenverdampfers 1 bis 50 theoretische Böden aufweist und oberhalb des Zwischenverdampfers 1 bis 200 theoretische Böden aufweist. Insbesondere ist es bevorzugt, wenn die Reaktionskolonne **RR_{A}** bzw. **RR_{B}** dann unterhalb des Zwischenverdampfers 2 bis 10 theoretische Böden aufweist und oberhalb des Zwischenverdampfers 20 bis 50 theoretische Böden aufweist.

Wenn die Reaktionskolonne **RR_{A}** bzw. Reaktionskolonne **RR_{B}** einen Zwischenverdampfer **VZ_{3A}** bzw. **VZ_{3B}** aufweist, ist es außerdem vorteilhaft, wenn der Seitenabzug (also die "Entnahmestelle **E_{RRA}"** an der Reaktionskolonne **RR_{A}** bzw. die "Entnahmestelle **E_{RRB}"** an der Reaktionskolonne **RR_{B}**), über den das Rohprodukt **RP_{A}** bzw. **RP_{B}** dem Zwischenverdampfer **VZ_{3A}** bzw. **VZ_{3B}** zugeführt wird, und der Seitenzulauf (also die "Zugabestelle **Z_{RRA}"** an der Reaktionskolonne **RR_{A}** bzw. die "Zugabestelle **Z_{RRB}"** an der Reaktionskolonne **RR_{B}**), über den das verdampfte Rohprodukt **RP_{A}** bzw. **RP_{B}** aus dem Zwischenverdampfer **VZ_{3A}** bzw. **VZ_{3B}** wieder der jeweiligen Reaktionskolonne **RR_{A}** bzw. **RR_{B}** zugeführt wird, zwischen den gleichen Böden der Reaktionskolonne **RR_{A}** bzw. Reaktionskolonne **RR_{B}** positioniert ist. Es ist jedoch auch möglich, dass Seitenabzug und Seitenzulauf auf unterschiedlicher Höhe liegen.

In einer bevorzugten Ausführungsform ist bei Einsatz eines Zwischenverdampfers **VZ_{3A}** bzw. **VZ_{3B}** in **RR_{A}** bzw. **RR_{B}** der Durchmesser der Reaktionskolonne **RR_{A}** bzw. **RR_{B}** oberhalb des Zwischenverdampfers **RR_{A}** bzw. **RR_{B}** größer als der Durchmesser der Reaktionskolonne **RR_{A}** bzw. **RR_{B}** unterhalb des Zwischenverdampfers **VZ_{3A}** bzw. **VZ_{3B}.** Dies hat den Vorteil, dass Investitionskosten gespart werden können.

In einem solchen Zwischenverdampfer **VZ_{3A}** bzw. **VZ_{3B}** kann in der Reaktionskolonne **RR_{A}** befindliches, flüssiges Rohprodukt **RP_{A}** umfassend M_{A}OR, Wasser, ROH, M_{A}OH bzw. in der Reaktionskolonne **RR_{B}** befindliches, flüssiges Rohprodukt **RP_{B}** umfassend M_{B}OR, Wasser, ROH, M_{B}OH in den gasförmigen Zustand überführt bzw., wenn es schon gasförmig voliegt, weiter erhitzt werden, und so die Effizienz der Umsetzung gemäß Schritt (a1) bzw. (a2) des erfindungsgemäßen Verfahrens verbessert werden.

Durch die Anordnung von einem oder mehreren Zwischenverdampfern **VZ_{3A}** im oberen Bereich der Reaktionskolonne **RR_{A}** bzw. von einem oder mehreren Zwischenverdampfern **VZ_{3B}** im oberen Bereich der Reaktionskolonne **RR_{B}** können die Abmessungen im unteren Bereich der Reaktionskolonne **RR_{A}** bzw. **RR_{B}** verringert werden. Bei der Ausführungsform mit mindestens einem, bevorzugt mehreren Zwischenverdampfern **VZ_{3A}** bzw. **VZ_{3B}** ist es auch möglich, Teilströme des ROH in flüssiger Form im oberen Bereich der Reaktionskolonne **RR_{A}** bzw. **RR_{B}** zuzuführen.

Sumpfverdampfer sind erfindungsgemäß am Sumpf der Reaktionskolonne **RR_{A}** bzw. **RR_{B}** angeordnet und werden dann als **"VS_{3A}"** bzw. **"VS_{3B}"** bezeichnet. In einem solchen Sumpfverdampfer kann in der Reaktionskolonne **RR_{A}** bzw. **RR_{B}** befindlicher Sumpfproduktstrom **S_{AP}** bzw. **S_{BP}** geleitet und ROH mindestens teilweise daraus entfernt werden, wodurch ein Sumpfproduktstrom **S_{AP*}** mit einem gegenüber **S_{AP}** erhöhten Massenanteil an M_{A}OR erhalten wird bzw. wodurch ein Sumpfproduktstrom **S_{BP*}** mit einem gegenüber **S_{BP}** erhöhten Massenanteil an M_{B}OR erhalten wird.

In Schritt (a1) des erfindungsgemäßen Verfahrens wird am unteren Ende der Reaktionskolonne **RR_{A}** ein Sumpfproduktstrom **S_{AP}** umfassend ROH und M_{A}OR entnommen.

Es ist bevorzugt, dass die Reaktionskolonne **RR_{A}** mindestens einen Sumpfverdampfer **VS_{3A}** aufweist, über den Sumpfproduktstrom **S_{AP}** dann mindestens teilweise geleitet wird und ROH aus diesem mindestens teilweise entfernt wird, wodurch ein Sumpfproduktstrom **S_{AP*}** mit einem gegenüber **S_{AP}** erhöhten Massenanteil an M_{A}OR erhalten wird.

Der Massenanteil an M_{A}OR des Sumpfproduktstroms **S_{AP*}** ist dabei insbesondere gegenüber dem Massenanteil an M_{A}OR des Sumpfproduktstroms **S_{AP}** um mindestens 1 % erhöht, bevorzugt um ≥ 2 %, bevorzugter um ≥ 5 %, noch bevorzugter um ≥ 10 %, noch mehr bevorzugter um ≥ 20 %, noch mehr bevorzugter um ≥ 30 %, noch mehr bevorzugter um ≥ 40 %, noch mehr bevorzugter um ≥ 50 %, noch mehr bevorzugter um ≥ 100 %, noch mehr bevorzugter um ≥ 150 %.

Bevorzugt weist **S_{AP}** oder, falls mindestens einen Sumpfverdampfer **VS_{3A}** eingesetzt wird, über den der Sumpfproduktstrom **S_{AP}** mindestens teilweise geleitet wird und ROH aus diesem mindestens teilweise entfernt wird, **S_{AP*},** einen Massenanteil von M_{A}OR in ROH im Bereich 1 bis 50 Gew.-%, bevorzugt 5 bis 32 Gew.-%, bevorzugter 15 bis 32 Gew.-%, am bevorzugtesten 30 bis 32 Gew.-% auf, jeweils bezogen auf die Gesamtmasse von **S_{AP}** bzw. **S_{AP*}.**

Der Massenanteil an Restwasser in **S_{AP}** bzw. **S_{AP*}** liegt dabei bevorzugt bei < 1 Gew.-%, bevorzugt < 0.1 Gew.-%, bevorzugter < 0.01 Gew.-%, bezogen auf die Gesamtmasse von **S_{AP}** bzw. **S_{AP*}.**

Der Massenanteil an Edukt M_{A}OH in **S_{AP}** bzw. **S_{AP*}** liegt dabei bevorzugt bei < 1 Gew.-%, bevorzugt < 0.1 Gew.-%, bevorzugter < 0.01 Gew.-%, bezogen auf die Gesamtmasse von **S_{AP}** bzw. **S_{AP*}.**

### 4.2 Schritt (a2) des erfindungsgemäßen Verfahrens (optional)

Schritt (a2) wird erfindungsgemäß durchgeführt oder auch nicht durchgeführt. Im optionalen Schritt (a2), der gleichzeitig mit und räumlich getrennt von Schritt (a1) des erfindungsgemäßen Verfahrens abläuft, wird ein Eduktstrom **S_{BE1}** umfassend ROH mit einem Eduktstrom **S_{BE2}** umfassend M_{B}OH im Gegenstrom bei einem Druck **p_{3B}** und einer Temperatur **T_{3B}** in einer Reaktivrektifikationskolonne **RR_{B}** zu einem Rohprodukt **RP_{B}** umfassend M_{B}OR, Wasser, ROH, M_{B}OH umgesetzt.

Im optionalen Schritt (a2) des erfindungsgemäßen Verfahrens wird am unteren Ende von **RR_{B}** ein Sumpfproduktstrom **S_{BP}** umfassend ROH und M_{B}OR entnommen. Am oberen Ende von **RR_{B}** wird ein Brüdenstrom **S_{BB}** umfassend Wasser und ROH entnommen.

M_{B} ist ausgewählt aus Natrium, Kalium, und bevorzugt Kalium.

Der Eduktstrom **S_{BE1}** umfasst ROH. In einer bevorzugten Ausführungsform liegt der Massenanteil von ROH in **S_{BE1},** bezogen auf die gesamte Masse des Eduktstroms **S_{BE1},** bei ≥ 95 Gew.-%, noch bevorzugter bei ≥ 99 Gew.-%, wobei **S_{BE1}** ansonsten insbesondere Wasser aufweist.

Der im optionalen Schritt (a2) des erfindungsgemäßen Verfahrens als Eduktstrom **S_{BE1}** eingesetzte Alkohol ROH kann auch handelsüblicher Alkohol mit einem Alkoholmassenanteil, bezogen auf die gesamte Masse des Eduktstroms **S_{BE1},** von mehr als 99.8 Gew.-% und einem Massenanteil an Wasser, bezogen auf die gesamte Masse des Eduktstroms **S_{BE1},** von bis zu 0.2 Gew.-% sein.

Der Eduktstrom **S_{BE1}** wird bevorzugt dampfförmig zugegeben.

Der Eduktstrom **S_{BE2}** umfasst M_{B}OH. In einer bevorzugten Ausführungsform umfasst **S_{BE2}** neben M_{B}OH mindestens eine weitere Verbindung ausgewählt aus Wasser, ROH. Noch bevorzugter umfasst **S_{BE2}** neben M_{B}OH Wasser, dann handelt es sich bei **S_{BE2}** um eine wässrige Lösung von M_{B}OH.

Wenn der Eduktstrom **S_{BE2}** M_{B}OH und Wasser umfasst, liegt der Massenanteil von M_{B}OH, bezogen auf das Gesamtgewicht des Eduktstroms **S_{BE2},** insbesondere im Bereich von 10 bis 55 Gew.-%, bevorzugt von 15 bis 54 Gew.-%, bevorzugter von 30 bis 53 Gew.-% und besonders bevorzugt von 45 bis 52 Gew.-%, am bevorzugtesten bei 50 Gew.-%.

Wenn der Eduktstrom **S_{BE2}** M_{B}OH und ROH umfasst, liegt der Massenanteil von M_{B}OH, bezogen auf das Gesamtgewicht des Eduktstroms **S_{BE2},** insbesondere im Bereich von 10 bis 55 Gew.-%, bevorzugt von 15 bis 54 Gew.-%, bevorzugter von 30 bis 53 Gew.-%, und besonders bevorzugt von 45 bis 52 Gew.-%.

In dem besonderen Fall, in dem der Eduktstrom **S_{BE2}** neben M_{B}OH sowohl Wasser als auch ROH umfasst, ist es besonders bevorzugt, dass der Massenanteil von M_{B}OH, bezogen auf das Gesamtgewicht des Eduktstroms **S_{BE2},** insbesondere im Bereich von 10 bis 55 Gew.-%, bevorzugt von 15 bis 54 Gew.-%, bevorzugter von 30 bis 53 Gew.-%, und besonders bevorzugt von 45 bis 52 Gew.-% liegt.

Schritt (a2) des erfindungsgemäßen Verfahrens wird in einer Reaktivrektifikationskolonne (oder "Reaktionskolonne") **RR_{B}** durchgeführt. Bevorzugte Ausführungsformen der Reaktionskolonne **RR_{B}** sind im Abschnitt 4.1 beschrieben.

"Umsetzung eines Eduktstroms **S_{BE1}** umfassend ROH mit einem Eduktstrom **S_{BE2}** umfassend M_{B}OH im Gegenstrom" wird erfindungsgemäß insbesondere dadurch gewährleistet, dass der Zugabepunkt mindestens eines Teils des Eduktstroms **S_{BE1}** umfassend ROH im optionalen Schritt (a2) an der Reaktionskolonne **RR_{B}** unterhalb des Zugabepunktes des Eduktstroms **S_{BE2}** umfassend M_{B}OH liegt.

Die Reaktionskolonne **RR_{B}** umfasst vorzugsweise mindestens 2, insbesondere 15 bis 40 theoretische Böden zwischen dem Zugabepunkt des Eduktstroms **S_{BE1}** und dem Zugabepunkt des Eduktstroms **S_{BE2}.**

Die Reaktionskolonne **RR_{B}** wird bevorzugt als reine Abtriebskolonne betrieben. Insbesondere wird demnach im unteren Bereich der Reaktionskolonne **RR_{B}** dampfförmig der Eduktstrom **S_{BE1}** umfassend ROH zugeführt. Der optionale Schritt (a2) des erfindungsgemäßen Verfahrens umfasst auch den Fall, dass ein Teil des Eduktstroms **S_{BE1}** umfassend ROH zwar unterhalb des Zugabepunktes des Eduktstroms **S_{BE2}** umfassend Alkalilauge M_{B}OH, aber dennoch am oberen Ende oder im Bereich des oberen Endes der Reaktionskolonne **RR_{B}** dampfförmig zugegeben wird. Dadurch können die Abmessungen im unteren Bereich der Reaktionskolonne **RR_{B}** verringert werden. Wenn ein Teil des Eduktstroms **S_{BE1}** umfassend ROH, insbesondere Methanol, am oberen Ende oder im Bereich des oberen Endes der Reaktionskolonne **RR_{B}** insbesondere dampfförmig zugegeben wird, so wird nur eine Teilmenge von insbesondere 10 bis 70 Gew.-%, bevorzugt von 30 bis 50 Gew.-% (jeweils bezogen auf die Gesamtmenge des im optionalen Schritt (a2) eingesetzten Alkohols ROH) am unteren Ende der Reaktionskolonne **RR_{B}** eingespeist und die restliche Teilmenge in einem Einzelstrom oder auf mehrere Teilströme verteilt, bevorzugt 1 bis 10 theoretische Böden, besonders bevorzugt 1 bis 3 theoretische Böden unterhalb des Zugabepunktes des Eduktstroms **S_{BE2}** umfassend M_{B}OH dampfförmig zugegeben.

In der Reaktionskolonne **RR_{B}** setzt sich dann der Eduktstrom **S_{BE1}** umfassend ROH mit dem Eduktstrom **S_{BE2}** umfassend M_{B}OH gemäß der vorstehend beschriebenen Reaktion <1> zu M_{B}OR und H₂O um, wobei, da es sich um eine Gleichgewichtsreaktion handelt, diese Produkte in Mischung mit den Edukten ROH und M_{B}OH vorliegen. Demnach wird im optionalen Schritt (a2) des erfindungsgemäßen Verfahrens in der Reaktionskolonne **RR_{B}** ein Rohprodukt **RP_{B}** erhalten, welches neben den Produkten M_{B}OR und Wasser auch noch ROH und M_{B}OH umfasst.

Am unteren Ende von **RR_{B}** erhält und entnimmt man dann den Sumpfproduktstrom **S_{BP}** umfassend ROH und M_{B}OR.

Am oberen Ende von **RR_{B},** bevorzugt am Kopf von **RR_{B},** entnimmt man noch Wasser enthaltenden Alkoholstrom, vorstehend als "Brüdenstrom **S_{BB}** umfassend Wasser und ROH" bezeichnet.

Dieser Brüdenstrom **S_{BB}** umfassend Wasser und ROH wird dem Schritt (b) des erfindungsgemäßen Verfahrens zugeführt. Dabei wird er, bevor er dem Schritt (b) des erfindungsgemäßen Verfahrens zugeführt wird, mit **S_{AB}** vermischt oder nicht, also getrennt von **S_{AB}** dem Schritt (b) des erfindungsgemäßen Verfahrens zugeführt. Bevorzugt wird Brüdenstrom **S_{BB}** mit **S_{AB}** vermischt und dann der erhaltene Mischbrüdenstrom dem Schritt (b) des erfindungsgemäßen Verfahrens zugeführt.

Die Menge des vom Eduktstrom **S_{BE1}** umfassten Alkohols ROH wird vorzugsweise so gewählt, dass dieser gleichzeitig als Lösungsmittel für das im Sumpfproduktstrom **S_{BP}** erhaltene Alkalialkoholat M_{B}OR dient. Vorzugsweise wird die Menge des Alkohols ROH im Eduktstrom **S_{BE1}** so gewählt, dass im Sumpf der Reaktionskolonne die gewünschte Konzentration der Alkalialkoholat-Lösung vorliegt, die als Sumpfproduktstrom **S_{BP}** umfassend ROH und M_{B}OR entnommen wird.

In einer bevorzugten Ausführungsform des optionalen Schritts (a2) des erfindungsgemäßen Verfahrens, und insbesondere in den Fällen, in denen **S_{BE2}** neben M_{B}OH auch Wasser umfasst, beträgt das Verhältnis aus dem Gesamtgewicht (Massen; Einheit: kg) an in Schritt (a2) als Eduktstrom **S_{BE1}** eingesetztem Alkohol ROH zum Gesamtgewicht (Massen; Einheit: kg) an in Schritt (a2) als Eduktstrom **S_{BE2}** eingesetzten M_{B}OH 1 : 1 bis 50 : 1, bevorzugter 5 : 1 bis 48 : 1, noch bevorzugter 9 : 1 bis 35 : 1, noch mehr bevorzugter 10 : 1 bis 30 : 1, noch mehr bevorzugter 13 : 1 bis 22 : 1, am bevorzugtesten 14 : 1.

Die Reaktionskolonne **RR_{B}** wird mit oder ohne, vorzugsweise ohne Rücklauf betrieben.

"Ohne Rücklauf" bedeutet, dass der am oberen Ende von **RR_{B}** entnommene Brüdenstrom **S_{BB}** umfassend Wasser und ROH der Rektifikationskolonne **RD₁** gemäß Schritt (b) vollständig zugeführt wird. Der Brüdenstrom **S_{BB}** umfassend Wasser und ROH wird der Rektifikationskolonne **RD₁** dabei vorzugsweise dampfförmig zugeführt.

"Mit Rücklauf" bedeutet, dass der am oberen Ende der jeweiligen Kolonne, in Schritt (a2) der Reaktionskolonne **RR_{B},** entnommene Brüdenstrom **S_{BB}** umfassend Wasser und ROH nicht vollständig abgeführt wird, in Schritt (b) also nicht vollständig der ersten Rektifikationskolonne **RD₁** zugeführt wird, sondern mindestens teilweise, bevorzugt teilweise, wieder als Rücklauf der jeweiligen Kolonne, in Schritt (a2) also der Reaktionskolonne **RR_{B},** zugeführt wird. In den Fällen, in denen ein solcher Rücklauf eingestellt wird, beträgt das Rücklaufverhältnis dabei bevorzugt 0.05 bis 0.99, bevorzugter 0.1 bis 0.9, noch bevorzugter 0.11 bis 0.34, besonderes bevorzugt 0.14 bis 0.27 und ganz besonders bevorzugt 0.17 bis 0.24. Ein Rücklauf kann dadurch eingestellt werden, dass am Kopf der jeweiligen Kolonne, in Schritt (a2) der Reaktionskolonne **RR_{B},** ein Kondensator **K_{RRB}** angebracht wird, in welchem der Brüdenstrom **S_{BB}** mindestens teilweise kondensiert wird und der jeweiligen Kolonne, in Schritt (a2) der Reaktionskolonne **RR_{B},** wieder zugeführt wird.

In der Ausführungsform, in der an der Reaktionskolonne **RR_{B}** ein Rücklauf eingestellt wird, kann der im optionalen Schritt (a2) als Eduktstrom **S_{BE2}** eingesetzte Alkohol M_{B}OH auch mindestens teilweise, bevorzugt teilweise, mit dem Rücklaufstrom vermischt werden und die resultierende Mischung so dem Schritt (a2) zugeführt werden.

Der optionale Schritt (a2) des erfindungsgemäßen Verfahrens wird insbesondere bei einer Temperatur **T_{3B}** im Bereich von 25 °C bis 200 °C, bevorzugt im Bereich von 45 °C bis 150 °C, bevorzugter im Bereich von 47 °C bis 120 °C, bevorzugter im Bereich von 60 °C bis 110 °C, durchgeführt.

Der optionale Schritt (a2) des erfindungsgemäßen Verfahrens wird insbesondere bei einem Druck **p_{3B}** von 0.5 bar bis 40 bar, bevorzugt im Bereich von 0.75 bar bis 5 bar, bevorzugter im Bereich von 1 bar bis 2 bar, bevorzugter im Bereich von 1 bar bis 1.8 bar, noch bevorzugter bei 1.1 bar bis 1.6 bar durchgeführt. Dabei ist erfindungswesentlich, dass bei der Einstellung des Druckes **p_{3B}** gilt: **p₁** > **p_{3B}.** Insbesondere gilt außerdem **p_{3B}** > **p₂.**

Die Reaktionskolonne **RR_{B}** umfasst in einer bevorzugten Ausführungsform mindestens einen Verdampfer, der insbesondere aus Zwischenverdampfern **VZ_{B}** und Sumpfverdampfern **VS_{B}** ausgewählt ist. Die Reaktionskolonne **RR_{B}** umfasst besonders bevorzugt mindestens einen Sumpfverdampfer **VS_{3B}.**

Im optionalen Schritt (a2) des erfindungsgemäßen Verfahrens wird am unteren Ende der Reaktionskolonne **RR_{B}** ein Sumpfproduktstrom **S_{BP}** umfassend ROH und M_{B}OR entnommen.

Es ist bevorzugt, dass die Reaktionskolonne **RR_{B}** mindestens einen Sumpfverdampfer **VS_{3B}** aufweist, über den Sumpfproduktstrom **S_{BP}** dann mindestens teilweise geleitet wird und ROH aus diesem mindestens teilweise entfernt wird, wodurch ein Sumpfproduktstrom **S_{BP*}** mit einem gegenüber **S_{BP}** erhöhten Massenanteil an M_{B}OR erhalten wird.

Der Massenanteil an M_{B}OR des Sumpfproduktstroms **S_{BP*}** ist dabei insbesondere gegenüber dem Massenanteil an M_{B}OR des Sumpfproduktstroms **S_{BP}** um mindestens 1 % erhöht, bevorzugt um ≥ 2 %, bevorzugter um ≥ 5 %, noch bevorzugter um ≥ 10 %, noch mehr bevorzugter um ≥ 20 %, noch mehr bevorzugter um ≥ 30 %, noch mehr bevorzugter um ≥ 40 %, noch mehr bevorzugter um ≥ 50 %, noch mehr bevorzugter um ≥ 100 %, noch mehr bevorzugter um ≥ 150 %.

Bevorzugt weist **S_{BP}** oder, falls mindestens einen Sumpfverdampfer **VS_{3B}** eingesetzt wird, über den der Sumpfproduktstrom **S_{BP}** mindestens teilweise geleitet wird und ROH aus diesem mindestens teilweise entfernt wird, **S_{BP*},** einen Massenanteil von M_{B}OR in ROH im Bereich 1 bis 50 Gew.-%, bevorzugt 5 bis 32 Gew.-%, bevorzugter 10 bis 32 Gew.-%, am bevorzugtesten 15 bis 30 Gew.-% auf, jeweils bezogen auf die Gesamtmasse von **S_{BP}** bzw. **S_{BP*}.**

Der Massenanteil an Restwasser in **S_{BP}** bzw. **S_{BP*}** liegt dabei bevorzugt bei < 1 Gew.-%, bevorzugt < 0.1 Gew.-%, bevorzugter < 0.01 Gew.-%, bezogen auf die Gesamtmasse von **S_{BP}** bzw. **S_{BP*}.**

Der Massenanteil an Edukt M_{B}OH in **S_{BP}** bzw. **S_{BP*}** liegt dabei bevorzugt bei < 1 Gew.-%, bevorzugt < 0.1 Gew.-%, bevorzugter < 0.01 Gew.-%, bezogen auf die Gesamtmasse von **S_{BP}** bzw. **S_{BP*}.**

In den Ausführungsformen des vorliegenden Verfahrens, in denen auch Schritt (a2) ausgeführt wird, wird bevorzugt der Sumpfproduktstrom **S_{AP}** mindestens teilweise über einen Sumpfverdampfer **VS_{3A}** geleitet und ROH aus **S_{AP}** mindestens teilweise entfernt, wodurch ein Sumpfproduktstrom **S_{AP*}** mit einem gegenüber **S_{AP}** erhöhten Massenanteil an M_{A}OR erhalten wird und/oder, bevorzugt und, der Sumpfproduktstrom **S_{BP}** mindestens teilweise über einen Sumpfverdampfer **VS_{3B}** geleitet und ROH aus **S_{BP}** mindestens teilweise entfernt, wodurch ein Sumpfproduktstrom **S_{BP*}** mit einem gegenüber **S_{BP}** erhöhten Massenanteil an M_{B}OR erhalten wird.

Der Schritt (a2) des erfindungsgemäßen Verfahrens wird in den Ausführungsformen der vorliegenden Erfindung, in denen er durchgeführt wird, gleichzeitig mit und räumlich getrennt von Schritt (a1) durchgeführt. Die räumliche Trennung wird durch die Durchführung der Schritte (a1) und (a2) in den beiden Reaktionskolonnen **RR_{A}** und **RR_{B}** gewährleistet.

In einer vorteilhaften Ausgestaltung der Erfindung sind die Reaktionskolonnen **RR_{A}** und **RR_{B}** in einem Kolonnenmantel untergebracht, wobei die Kolonne mindestens teilweise durch mindestens eine Trennwand unterteilt ist. Eine solche mindestens eine Trennwand aufweisende Kolonne wird erfindungsgemäß als **"TRD"** bezeichnet. Solche Trennwandkolonnen sind dem Fachmann bekannt und beispielsweise beschrieben in US 2,295,256, EP 0 122 367 A2, EP 0 126 288 A2, WO 2010/097318 A1 sowie von I. Dejanović, Lj. Matijašević, Ž. Olujić, *Chemical Engineering and Processing* **2010,** *49*, 559-580. In den für das erfindungsgemäße Verfahren in Frage kommenden Trennwandkolonnen sind die Trennwände bevorzugt bis zum Boden durchgezogen und durchspannen insbesondere bevorzugt mindestens ein Viertel, bevorzugter mindestens ein Drittel, noch bevorzugter mindestens die Hälfte, noch bevorzugter mindesten zwei Drittel, noch mehr bevorzugter mindestens drei Viertel der Kolonne der Länge nach. Sie teilen die Kolonne in mindestens zwei Reaktionsräume, in denen räumlich getrennte Reaktionen stattfinden können. Die durch die mindestens eine Trennwand geschaffenen Reaktionsräume können gleich oder unterschiedlich groß sein.

In dem jeweils von der Trennwand getrennten Bereichen können in dieser Ausführungsform die Sumpfproduktströme **S_{AP}** und **S_{BP}** getrennt entnommen werden und bevorzugt über den für jeden durch die mindestens eine Reaktionswand gebildeten Reaktionsraum angebrachten Sumpfverdampfer **VS_{3A}** bzw. **VS_{3B}** geleitet werden , in dem ROH aus **S_{AP}** bzw. **S_{BP}** mindestens teilweise entfernt wird, wodurch **S_{AP*}** bzw. **S_{BP*}** erhalten werden.

### 4.3 Schritt (b) des erfindungsgemäßen Verfahrens

Im Schritt (b) des erfindungsgemäßen Verfahrens wird der Brüdenstrom **S_{AB},** und, wenn Schritt (a2) durchgeführt wird, der Brüdenstrom **S_{BB},** vermischt mit **S_{AB}** oder getrennt von **S_{AB},** in eine erste Rektifikationskolonne **RD₁** geleitet,
wodurch ein Gemisch **G_{RD1}** umfassend Wasser und ROH in der Rektifikationskolonne **RD₁** erhalten wird.

In der optionalen Ausführung des erfindungsgemäßen Verfahrens, in der Schritt (a2) durchgeführt wird, wird der Brüdenstrom **S_{BB}** dabei bevorzugt vermischt mit **S_{AB}** und dann der erhaltene Mischbrüden **S_{ABB}** in eine Rektifikationskolonne **RD₁** geleitet.

Der Brüdenstrom **S_{AB}** und, in den Fällen, in denen der optionale Schritt (a2) durchgeführt wird, der Brüdenstrom **S_{BB},** können in einer Ausführungsform der vorliegenden Erfindung (wenn **p_{3A}** < **p₁** bzw. **p_{3B}** < **p₁)** verdichtet werden, bevor sie in die Rektifikationskolonne **RD₁** geleitet werden. Dies kann über einen Verdichter **VD₃₁** geschehen. In den Ausführungsformen der vorliegenden Erfindung, in denen **p_{3A} > p₁** und **p_{3B}** > **p₁,** ist die Bereitstellung eines Verdichters **VD₃₁** jedoch nicht nötig, und die Bereitstellung eines solchen und somit die dazu nötige elektrische Energie können dann eingespart werden.

Es versteht sich von selbst, dass auch in den Ausführungsformen, in denen der optionale Schritt (a2) durchgeführt wird, und **S_{BB}** getrennt von **S_{AB}** in die Rektifikationskolonne **RD₁** geleitet wird, sich **S_{AB}** und **S_{BB}** in der Rektifikationskolonne **RD₁** vermischen, so dass in jedem Fall nach Durchführung von Schritt (b) ein Gemisch **G_{RD1}** umfassend Wasser und ROH in der ersten Rektifikationskolonne **RD₁** erhalten wird.

Als Rektifikationskolonne **RD₁** in Schritt (b) des erfindungsgemäßen Verfahrens kann jede beliebige, dem Fachmann bekannte Rektifikationskolonne eingesetzt werden. Bevorzugt enthält die Rektifikationskolonne **RD₁** Einbauten. Geeignete Einbauten sind zum Beispiel Böden, unstrukturierte Packungen oder strukturierte Packungen. Als Böden werden üblicherweise Glockenböden, Siebböden, Ventilböden, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel oder Intalox^{®}-Sättel verwendet. Strukturierte Packungen werden zum Beispiel unter dem Handelsnamen Mellapack^{®} der Firma Sulzer vertrieben. Neben den genannten Einbauten weitere geeignete Einbauten sind dem Fachmann bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Rektifikationskolonne möglichst gering bleibt und somit die mechanische Leistung des Verdichters und die Temperatur des zu verdampfenden Alkohol/Wasser-Gemischs **G_{RD1}** gering bleibt.

Wenn in der Rektifikationskolonne **RD₁** strukturierte Packungen oder unstrukturierte Packungen enthalten sind, so können diese geteilt sein oder es kann eine durchgehende Packung vorliegen. Üblicherweise sind jedoch mindestens zwei Packungen vorgesehen, eine Packung oberhalb der Zulaufstelle des Brüdenstroms **S_{AB}** bzw. der Zulaufstellen der beiden Brüdenströme **S_{AB}** und **S_{BB}** und eine Packung unterhalb der Zulaufstelle des Brüdenstroms **S_{AB}** bzw. der Zulaufstellen der beiden Brüdenströme **S_{AB}** und **S_{BB}** bzw. der Zulaufstelle des Mischbrüden **S_{ABB}.** Wenn eine unstrukturierte Packung eingesetzt wird, beispielsweise eine Füllkörperpackung, so liegen die Füllkörper üblicherweise auf einem geeigneten Siebboden oder Gitterboden auf.

Am Ende des Schrittes (b) des erfindungsgemäßen Verfahrens wird schließlich ein Gemisch **G_{RD1}** umfassend Wasser und ROH in der Rektifikationskolonne **RD₁** erhalten. Die Zusammensetzung des Gemisches **G_{RD1}** ergibt sich insbesondere aus der Zusammensetzung des Brüdenstroms **S_{AB}** bzw., wenn Schritt (a2) durchgeführt wird, insbesondere anteilig aus der Zusammensetzung der beiden Brüdenströme **S_{AB}** und **S_{BB}.**

### 4.4 Schritt (c) des erfindungsgemäßen Verfahrens

Im Schritt (c) des erfindungsgemäßen Verfahrens wird das Gemisch **G_{RD1}** umfassend Wasser und ROH in der ersten Rektifikationskolonne **RD₁** bei einem Druck **p₁** und einer Temperatur **T₁** in einen ROH umfassenden Brüdenstrom **S_{RDB1}** am oberen Ende (= Kopf) von **RD₁** und einen Sumpfstrom **S_{RDS1}** umfassend Wasser und ROH am unteren Ende (= Sumpf) von **RD₁** aufgetrennt.

Der Druck **p₁** in **RD₁** kann abgesehen davon, dass die Bedingung **p₁** > **p₂** gelten muss, vom Fachmann gemäß seinem Fachwissen eingestellt werden. Er liegt bevorzugt im Bereich zwischen 1 bar und 20 bar, bevorzugt 1 bar und 15 bar, bevorzugter 2 bis 14 bar, noch bevorzugter bei 4.00 bis 11.00 bar, noch bevorzugter bei 6.00 bis 10.00 bar, noch bevorzugter bei 7.00 bis 8.90 bar, wobei gleichzeitig jeweils gilt, dass **p₁** > **p₂.**

Die Temperatur **T₁** in **RD₁** kann vom Fachmann gemäß seinem Fachwissen eingestellt werden. Die liegt bevorzugt im Bereich von 40 °C bis 220 °C, bevorzugt 60 °C bis 190 °C.

In einer bevorzugten Ausführungsform ist **p_{3A}** > **p₂** und in den Fällen, in denen Schritt (a2) durchgeführt wird, zusätzlich **p_{3B} > p₂.** Durch diese Druckeinstellung wird der Gesamtenergiebedarf des Verfahrens gegenüber den Ausführungsformen, in denen **p_{3A} < p₂** bzw. **p_{3B}** < **p₂,** überraschend minimiert.

Bei der Auftrennung gemäß Schritt (c) des erfindungsgemäßen Verfahrens handelt es sich um eine dem Fachmann bekannte destillative Auftrennung des Alkohol-Wasser-Gemischs **G_{RD1}.**

Am unteren Ende (andere Bezeichnung: "Sumpf`) der Rektifikationskolonne **RD₁** wird ein Sumpfstrom **S_{RDS1}** erhalten, der noch Alkohol ROH umfasst. **S_{RDS1}** umfasst ROH in einem Massenanteil von insbesondere 0.005 bis 95 Gew.-%, bevorzugt 25 bis 95 Gew.-%, bezogen auf die Gesamtmasse von **S_{RDS1}.** Bevorzugt umfasst **S_{RDS1}** neben dem Alkohol ROH im Wesentlichen Wasser.

**S_{RDB1}** wird in einer bevorzugten Ausführungsform der Erfindung mindestens teilweise als Eduktstrom **S_{AE1}** in der Reaktivrektifikationskolonne **RR_{A}** und, wenn Schritt (a2) durchgeführt wird, alternativ oder zusätzlich als Eduktstrom **S_{BE1}** in der Reaktivrektifikationskolonne **RR_{B}** eingesetzt.

Am Kopf der Rektifikationskolonne **RD₁** wird außerdem der Brüdenstrom **S_{RDB1}** umfassend ROH erhalten. Der bevorzugte Massenanteil von ROH in diesem Brüdenstrom **S_{RDB1}** ist ≥ 99 Gew.-%, bevorzugt ≥ 99.6 Gew.-%, bevorzugter ≥ 99.9 Gew.-%, jeweils bezogen auf die Gesamtmasse von **S_{RDB1},** wobei der Rest insbesondere Wasser ist.

In Schritt (c) erfolgt die destillative Trennung des in Schritt (a1) bzw. Schritt (a1) und (a2) erhaltenen Brüden **S_{AB}** bzw. **S_{AB}** und **S_{BB}.** Diese umfassen im Wesentlichen den Alkohol ROH und Wasser. Insbesondere handelt es sich bei **S_{AB}** bzw. **S_{AB}** und **S_{BB}** um eine Wasser-Alkohol-Mischung, in dem der Massenanteil von ROH bevorzugt im Bereich > 80 Gew.-%, bevorzugter > 85, noch bevorzugter > 90 Gew.-% (bezogen auf die Gesamtmasse von **S_{AB}** bzw. **S_{AB}** und **S_{BB})** liegt. Somit ist insbesondere auch **G_{RD1}** ein Alkohol-Wasser-Gemisch, in dem der Massenanteil von ROH bevorzugt im Bereich > 80 Gew.-%, bevorzugter > 95 Gew.-%, noch bevorzugter > 90 Gew.- % (bezogen auf die Gesamtmasse von **G_{RD1}**) liegt.

### 4.5 Schritt (d) des erfindungsgemäßen Verfahrens

Im Schritt (d) des erfindungsgemäßen Verfahrens wird der Sumpfstrom **S_{RDS1}** ganz oder teilweise, bevorzugt teilweise, in eine zweite Rektifikationskolonne **RD₂** geleitet.

Dadurch wird ein Gemisch **G_{RD2}** umfassend Wasser und ROH in der zweiten Rektifikationskolonne **RD₂** erhalten.

In der Ausführungsform der vorliegenden Erfindung, in der **S_{RDS1}** teilweise in **RD₂** geleitet wird, wird dies insbesondere so durchgeführt wird, dass ein erster Teil **S_{RDS11}** des aus der ersten Rektifikationskolonne **RD₁** geleiteten Sumpfstroms **S_{RDS1}** in eine zweiten Rektifikationskolonne **RD₂** geleitet wird, und ein zweiter Teil **S_{RDS12}** des aus der ersten Rektifikationskolonne **RD₁** geleiteten Sumpfstroms **S_{RDS1}** in die erste Rektifikationskolonne **RD₁** zurückgeleitet wird. Noch bevorzugter wird dabei Energie auf **S_{RDS12}** übertragen, noch viel bevorzugter wird **S_{RDS12}** beheizt. Nachdem **S_{RDS12}** in **RD₁** zurückgeleitet wurde, vermischt es sich in **RD₁** mit **G_{RD1}** und liefert so Energie zur Auftrennung von **G_{RD1}** gemäß Schritt (c).

In dieser bevorzugten Ausführungsform des Schrittes (d) des erfindungsgemäßen Verfahrens ist es noch bevorzugter, wenn das Verhältnis der Massen (in kg) von **S_{RDS11}** zu **S_{RDS12}** im Bereich 9 : 1 bis 1 :9, noch bevorzugter 4 :1 bis 1 : 4, noch bevorzugter 7 : 3 bis 3 : 7, noch bevorzugter 3 : 2 bis 2: 3, noch bevorzugter 1 : 1 liegt.

In dieser bevorzugten Ausführungsform des Schrittes (d) des erfindungsgemäßen Verfahrens ist es möglich, dem Strom **S_{RDS12}** Energie zuzuführen. In einer bevorzugten Ausführungsform geschieht dies dergestalt, dass der Strom **S_{RDS12}** über einen Sumpfverdampfer **VS_{RD1}** geleitet wird, in welchem Energie von einem Wärmemedium auf **S_{RDS12}** übertragen wird. Diese Energieübertragung kann vorteilhafterweise vorgenommen werden, in dem **S_{RDS12}** und das Wärmemedium über einen Sumpfverdampfer **VS_{RD1}** geleitet werden. **S_{RDS12}** überträgt dann, nach der Zurückleitung von **S_{RDS12}** in die Reaktionskolonne **RR_{A},** die Energie auf **G_{RD1}.**

Als Rektifikationskolonne **RD₂** in Schritt (d) des erfindungsgemäßen Verfahrens kann jede beliebige, dem Fachmann bekannte Rektifikationskolonne eingesetzt werden. Bevorzugt enthält die Rektifikationskolonne **RD₂** Einbauten. Geeignete Einbauten sind zum Beispiel Böden, unstrukturierte Packungen oder strukturierte Packungen. Als Böden werden üblicherweise Glockenböden, Siebböden, Ventilböden, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel oder Intalox^{®}-Sättel verwendet. Strukturierte Packungen werden zum Beispiel unter dem Handelsnamen Mellapack^{®} der Firma Sulzer vertrieben. Neben den genannten Einbauten weitere geeignete Einbauten sind dem Fachmann bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Rektifikationskolonne **RD₂** möglichst gering bleibt und somit die mechanische Leistung des Verdichters und die Temperatur des zu verdampfenden Alkohol/Wasser-Gemischs **G_{RD2}** gering bleibt.

Wenn in der Rektifikationskolonne **RD₂** strukturierte Packungen oder unstrukturierte Packungen enthalten sind, so können diese geteilt sein oder es kann eine durchgehende Packung vorliegen. Üblicherweise sind jedoch mindestens zwei Packungen vorgesehen, eine Packung oberhalb der Zulaufstelle des Stroms **S_{RDS1}** bzw. des Teils von **S_{RDS1},** insbesondere **S_{RDS12}** und eine Packung unterhalb der entsprechenden Zulaufstelle. Wenn eine unstrukturierte Packung eingesetzt wird, beispielsweise eine Füllkörperpackung, so liegen die Füllkörper üblicherweise auf einem geeigneten Siebboden oder Gitterboden auf.

**S_{RDS1}** bzw. der Teil von **S_{RDS1},** der in **RD₂** geleitet wird, und bei dem es sich bevorzugt um **S_{RDS12}** handelt, ist zumindest teilweise flüssig.

Deshalb ist es weiterhin bevorzugt, diese über einen Flüssigverdichter oder eine Pumpe P in **RD₂** zu leiten.

Am Ende des Schrittes (d) des erfindungsgemäßen Verfahrens wird schließlich ein Gemisch **G_{RD2}** umfassend Wasser und ROH in der Rektifikationskolonne **RD₂** erhalten. Die Zusammensetzung des Gemisches **G_{RD2}** ergibt sich insbesondere aus der Zusammensetzung des Stroms **S_{RDS1}** bzw. des Teiles des Stroms **S_{RDS1},** bevorzugt **S_{RDS12},** der in **RD₂** geleitet wird,

### 4.6 Schritt (e) des erfindungsgemäßen Verfahrens

Im Schritt (e) des erfindungsgemäßen Verfahrens wird das Gemisch **G_{RD2}** umfassend Wasser und ROH bei einem Druck **p₂** und einer Temperatur **T₂** in einen ROH umfassenden Brüdenstrom **S_{RDB2}** am Kopf von **RD₂** und einen Sumpfstrom **S_{RDS2}** umfassend Wasser und gegebenenfalls ROH am Sumpf von **RD₂** aufgetrennt.

Der Druck **p₂** in **RD₂** kann abgesehen davon, dass die Bedingung **p₁** > **p₂** gelten muss, vom Fachmann gemäß seinem Fachwissen eingestellt werden. Er liegt bevorzugt im Bereich zwischen 1 bar und 20 bar, bevorzugt 1 bar und 15 bar, bevorzugter 1 bis 10 bar, noch bevorzugter bei 1.00 bis 2.00 bar, noch bevorzugter bei 1.10 bis 1.80 bar, noch bevorzugter bei 1.10 bis 1.50 bar, wobei gleichzeitig jeweils gilt, dass **p₁** > **p₂.**

Die Temperatur **T₂** in **RD₂** kann vom Fachmann gemäß seinem Fachwissen eingestellt werden. Die liegt bevorzugt im Bereich von 40 °C bis 220 °C, bevorzugt 60 °C bis 190 °C.

Bei der Auftrennung gemäß Schritt (e) des erfindungsgemäßen Verfahrens handelt es sich um eine dem Fachmann bekannte destillative Auftrennung des Alkohol-Wasser-Gemischs **G_{RD2}.**

Am Sumpf der Rektifikationskolonne **RD₂** wird ein Strom **S_{RDS2}** erhalten, der < 1 Gew.-% Alkohol, bezogen auf die Gesamtmasse von **S_{RDS2},** aufweisen kann.

Am Kopf der Rektifikationskolonne **RD₂** wird außerdem der Brüdenstrom **S_{RDB2}** umfassend ROH erhalten. Der bevorzugte Massenanteil von ROH in diesem Brüdenstrom **S_{RDB2}** ist ≥ 99 Gew.-%, bevorzugt ≥ 99.6 Gew.-%, bevorzugter ≥ 99.9 Gew.-%, jeweils bezogen auf die Gesamtmasse von **S_{RDB2},** wobei der Rest insbesondere Wasser ist.

In einer bevorzugten Ausführungsform der vorliegenden Erfindung wird **S_{RDB2}** mindestens teilweise als Eduktstrom **S_{AE1}** in der Reaktivrektifikationskolonne **RR_{A}** und, wenn Schritt (a2) durchgeführt wird, alternativ oder zusätzlich als Eduktstrom **S_{BE1}** in der Reaktivrektifikationskolonne **RR_{B}** eingesetzt.

In Schritt (e) erfolgt die destillative Trennung des in Schritt (d) ganz oder teilweise in die zweite Rektifikationskolonne **RD₂** geleiteten Stromes **S_{RDS1},** bevorzugt des Teiles **S_{RDS12}.**

### 4.7 Druckmanagement als kennzeichnendes Merkmal

Das erfindungsgemäße Verfahren ist dadurch gekennzeichnet, dass beim Betrieb der Rektifikationskolonnen **RD₁** (Schritt (c)) und **RD₂** (Schritt (e)) ein bestimmtes Druckverhältnis eingestellt wird.

Demnach gilt, dass **p₁** > **p₂, p₁** > **p_{3A},** und in den Fällen, in denen Schritt (a2) durchgeführt wird, **p₁ > p_{3B}.**

Es wurde überraschend festgestellt, dass bei Einhaltung dieser Drücke, der Bedarf an als in Form von Heizdampf zuzuführender Energie minimiert werden kann und der Großteil der für das Verfahren nötigen Energie durch Strom gedeckt werden kann.

Noch vorteilhafter ist es, wenn zusätzlich die Drücke so eingestellt werden, dass **p_{3A} > p₂** gilt, und in den Fällen, in denen Schritt (a2) durchgeführt wird, zusätzlich **p_{3B}** > **p₂** gilt. Eine solche Einstellung der Drücke **p_{3A}** und **p_{3B}** senkt den insgesamt aufzuwendenden Energiebedarf gegenüber dem Fall, dass **p_{3A}** < **p₂** bzw. **p_{3B}** < **p₂.**

### 4.8 Kennzeichnender Schritt (f): Energieübertragung von S_{RDB2} auf G_{RD1}

Der neben dem Druckregime charakteristische Schritt (f) des erfindungsgemäßen Verfahrens ist, dass Energie von **S_{RDB1}** auf das Gemisch **G_{RD2}** in der zweiten Rektifikationskolonne **RD₂** übertragen wird. Unter "Energieübertragung" wird erfindungsgemäß insbesondere "Wärmeübertragung" verstanden.

Dieser Schritt (f) und das erfindungsgemäße Druckregime erlauben eine besonders vorteilhafte Integration der ansonsten dissipierenden Energie, wodurch es gelingt, einen besonders großen Teil des Energiebedarfs des Verfahrens durch Strom anstatt durch Heizdampf zu decken. Damit ist das erfindungsgemäße Verfahren besonders energieeffizient.

Die Übertragung der Energie von **S_{RDB1}** auf **G_{RD2}** in **RD₂** kann erfindungsgemäß über verschiedene, dem Fachmann geläufige Wege erfolgen und bedeutet bevorzugt die Beheizung von **G_{RD2}** in **RD₂** mit **S_{RDB1}**, z.B. über einen Wärmeüberträger **WT.**

Erfindungsgemäß wird in Schritt (f) die Energie von **S_{RDB1}** auf **G_{RD2}** in **RD₂** insbesondere direkt oder indirekt, bevorzugt direkt übertragen.

### 4.8.1 Direkte Energieübertragung von S_{RDB1} auf G_{RD2} in RD₂

"Direkte Energieübertragung von **S_{RDB1}** auf **G_{RD2}** in **RD₂**" bedeutet erfindungsgemäß, dass eine Energieübertragung, bevorzugt Beheizung, von **G_{RD2}** in **RD₂** mit **S_{RDB1}** so erfolgt, dass **G_{RD2}** mit **S_{RDB1}** kontaktiert wird, ohne dass sich **G_{RD2}** mit **S_{RDB1}** mischt, so dass Energie von **S_{RDB1}** auf **G_{RD2}** übergeht. Unter die Fälle der direkten Energieübertragung werden erfindungsgemäß aber auch Fälle kategorisiert, in denen eine Energieübertragung, bevorzugt Beheizung, eines aus **RD₂** geleiteten Stromes **S_{X}** mit **S_{RDB1}** erfolgt, ohne dass sich **S_{RDB1}** mit **S_{X}** mischt, so dass Energie von **S_{RDB1}** auf **S_{X}** übergeht, und **S_{X}** danach wieder in **RD₂** geleitet wird, wo es sich mit **G_{RD2}** in **RD₂** vermischt und so die von **S_{RDB1}** aufgenommene Energie auf **G_{RD2}** in **RD₂** überträgt.

**S_{X}** ist in einer besonderen Ausführungsform der vorliegenden Erfindung aus der Gruppe bestehend aus **S_{RDS22}**, **S_{RDX2}** ausgewählt.

Die Kontaktierung ohne Vermischung wird nach dem Fachmann bekannten Verfahren erreicht, zum Beispiel über die Kontaktierung über eine Trennwand aus Metall, Kunststoff etc. insbesondere im Wärmeüberträger **WT,** bevorzugt einem Kondensator **K** oder Verdampfer **V,** der insbesondere aus Sumpfverdampfern **VS** und Zwischenverdampfern **VZ** ausgewählt ist.

Bevorzugt wird erfindungsgemäß die direkte Energieübertragung von **S_{RDB1}** auf das Gemisch **G_{RD2}** in der zweiten Rektifikationskolonne **RD₂** nach mindestens einem der Schritte (α-i), (α-ii), (α-iii) durchgeführt, bevorzugter nach mindestens einem der Schritte (α-i), (α-ii) durchgeführt.

(α-i) Energie von **S_{RDB1}** wird auf einen Teil **S_{RDS22}** des aus **RD₂** geleiteten Sumpfstroms **S_{RDS2}** übertragen und **S_{RDS22}** dann in **RD₂** zurückgeleitet. Dieser Schritt (α-i) umfasst auch Ausführungsformen, in denen man zunächst Energie von **S_{RDB1}**, bevorzugt über einen Wärmeüberträger **WT,** auf den gesamten Sumpfstrom **S_{RDS2}** überträgt, und dann erst vom Sumpfstrom **S_{RDS2}** den Teil **S_{RDS22}** abtrennt, und danach **S_{RDS22}** in **RD₂** zurückleitet.

(α-ii) Mindestens ein von **S_{RDB2}** und **S_{RDS2}** verschiedener Strom **S_{RDX2}** umfassend ROH und Wasser wird aus **RD₂** geleitet, dann wird Energie von **S_{RDB1}** auf **S_{RDX2},** bevorzugt über einen Wärmeüberträger **WT,** übertragen und **S_{RDX2}** in **RD₂** zurückgeleitet.

Bevorzugt wird **S_{RDX2}** unterhalb des Brüdenstroms **S_{RDB2}** an **RD₂** entnommen. Insbesondere ist **S_{RDX2}** dann ausgewählt aus Sumpfstrom **S_{RDX2S},** Zwischenstrom **S_{RDX2Z}.**

Ein Sumpfstrom **S_{RDX2S}** ist ein Strom, dessen Entnahmestelle an **RD₂** auf gleicher Höhe wie die Entnahmestelle von **S_{RDS2}** oder darunter liegt. **S_{RDX2S}** kann dann durch einen Wärmeüberträger **WT,** insbesondere einen Sumpfverdampfer **VS** geleitet werden, und in diesem Energie von **S_{RDB1}** auf **S_{RDX2S}** übertragen werden.

Ein Zwischenstrom **S_{RDX2Z}** ist ein Strom, dessen Entnahmestelle an **RD₂** zwischen den Entnahmestellen von **S_{RDB2}** und **S_{RDS2}** liegt. **S_{RDX2Z}** kann dann aus **RD₂** und durch einen Wärmeüberträger **WT,** insbesondere einen Zwischenverdampfer **VZ** geleitet werden, und in diesem Energie von **S_{RDB1}** auf **S_{RDX2Z}** übertragen werden.

(α-iii) **S_{RDB1}** wird durch **RD₂** geleitet, wodurch Energie von **S_{RDB1}** auf **G_{RD2}** übertragen wird, bevorzugt über einen Wärmeüberträger **WT.** Eine solche Ausführungsform kann beispielsweise realisiert werden, in dem **S_{RDB1}** durch die Rektifikationskolonne **RD₂** durch eine Leitung geführt wird, über deren Oberfläche **S_{RDB1}** Energie auf **G_{RD2}** in **RD₂** überträgt.

### 4.8.2 Indirekte Energieübertragung von S_{RDB1} auf G_{RD2} in RD₂

"Indirekte Energieübertragung von **S_{RDB1}** auf **G_{RD2}** in **RD₂**" bedeutet erfindungsgemäß, dass eine Energieübertragung, bevorzugt Beheizung, von **G_{RD2}** in **RD₂** mit **S_{RDB1}** so erfolgt, dass **G_{RD2}** nicht direkt mit **S_{RDB1}** kontaktiert wird, sondern mindestens ein zusätzlicher, bevorzugt genau ein zusätzlicher, von **G_{RD2}** und **S_{RDB1}** verschiedener Wärmeträger **W₁** eingeschaltet wird, der sich bei der Energieübertragung von **S_{RDB1}** auf **G_{RD2}** in **RD₂** weder mit **S_{RDB1}** noch mit **G_{RD2}** in **RD₂** mischt. Dabei wird die Energie von **S_{RDB1}** auf den mindestens einen Wärmeträger **W₁** übertragen, ohne dass sich **S_{RDB1}** und der mindestens eine Wärmeträger **W₁** mischen, und dann von dem mindestens eine Wärmeträger **W₁** auf **G_{RD2}** in **RD₂** übertragen, ohne dass sich der mindestens eine Wärmeträger **W₁** und **G_{RD2}** mischen.

Unter die Fälle der indirekten Energieübertragung werden erfindungsgemäß auch Fälle kategorisiert, in denen Energie von **S_{RDB1}** auf den mindestens einen, bevorzugt genau einen, Wärmeträger **W₁** übertragen wird, ohne dass sich **S_{RDB1}** und der mindestens eine Wärmeträger **W₁** mischen, und danach eine Energieübertragung, bevorzugt Beheizung, eines aus **RD₂** geleiteten Stromes **S_{X}** mit der mindestens eine Wärmeträger **W₁** erfolgt, ohne dass sich der mindestens eine Wärmeträger **W₁** mit **S_{X}** mischt, so dass Energie vom mindestens einen Wärmeträger **W₁** auf **S_{X}** übergeht, und **S_{X}** danach wieder in **RD₂** geleitet wird, wo es sich mit **G_{RD2}** in **RD₂** vermischt und so die von **S_{RDB1}** über den mindestens einen Wärmeträger **W₁** aufgenommene Energie auf **G_{RD2}** in **RD₂** überträgt.

**S_{X}** ist in einer besonderen Ausführungsform der vorliegenden Erfindung aus der Gruppe bestehend aus **S_{RDS22}, S_{RDX2}** ausgewählt.

"Mindestens einen Wärmeträger **W₁**" umfasst die Fälle, in denen die Energie von **W₁** erst auf einen oder mehrere weitere, von **G_{RD2}** und **S_{RDB1}** verschiedene Wärmeträger **W₂, W₃, W₄, W₅** etc. übertragen wird und der letzte dieser Wärmeträger, bezeichnet als "**W_{Y}"**, mit **G_{RD2}** in **RD₁** kontaktiert wird, so dass Energie, bevorzugt Wärme, von **W_{Y}** auf **G_{RD2}** übergeht, und sich **W_{Y}** und **G_{RD2}** aber nicht mischen. Ebenso kann Energie, bevorzugt Wärme, von **W_{Y}** auf einen aus **RD₂** geleiteten Strom **S_{X}** übertragen werden, ohne dass sich **W_{Y}** und **S_{X}** mischen, und **S_{X}** danach wieder in **RD₂** geleitet werden, wo es sich mit **G_{RD2}** in **RD₂** vermischt und so die von **W_{Y}** aufgenommene Energie auf **G_{RD2}** in **RD₂** überträgt.

Die beschriebenen Kontaktierungen findet dabei jeweils bevorzugt in einem Wärmeübertrager **WT,** bevorzugt einem Kondensator **K** oder Verdampfer **V,** der insbesondere aus Sumpfverdampfern **VS** und Zwischenverdampfern **VZ** ausgewählt ist, statt.

Bevorzugt wird erfindungsgemäß die indirekte Energieübertragung von **S_{RDB1}** auf das Gemisch **G_{RD2}** in der zweiten Rektifikationskolonne **RD₂** nach mindestens einem der Schritte (β-i), (β-ii), (β-iii) durchgeführt, bevorzugter nach mindestens einem der Schritte (β-i), (β-ii) durchgeführt.

(β-i) Ein Teil **S_{RDS22}** des aus **RD₂** geleiteten Sumpfstroms **S_{RDS2}** wird in die zweite Rektifikationskolonne **RD₂** zurückgeleitet. Energie wird von **S_{RDB1}** auf mindestens einen von **S_{RDS22}** verschiedenen Wärmeträger **Wᵢ₁** übertragen und dann von dem mindestens einen Wärmeträger **Wᵢ₁** auf **S_{RDS22}** übertragen wird, und **S_{RDS22}** dann in **RD₂** zurückgeleitet wird;
Dieser Schritt (β-i) umfasst auch Ausführungsformen, in denen man zunächst Energie von dem mindestens einen, bevorzugt genau einen, von **S_{RDS22}** verschiedenen Wärmeträger **Wᵢ₁,** bevorzugt über einen Wärmeüberträger **WT,** auf den gesamten Sumpfstrom **S_{RDS2}** überträgt, und dann erst vom Sumpfstrom **S_{RDS2}** den Teil **S_{RDS22}** abtrennt, und danach **S_{RDS22}** in **RD₂** zurückleitet.

(β-ii) Mindestens ein von **S_{RDB2}** und **S_{RDS2}** verschiedener Strom **S_{RDX2}** umfassend ROH und Wasser wird aus **RD₂** geleitet. Es wird Energie von **S_{RDB1}** auf mindestens einen, bevorzugt genau einen, von **S_{RDX2}** verschiedenen Wärmeträger **Wᵢᵢ₁** übertragen, bevorzugt über einen Wärmeüberträger **WT,** und dann von dem mindestens einen Wärmeträger **Wᵢᵢ₁** auf **S_{RDX2}** übertragen, dann wird **S_{RDX2}** in **RD₂** zurückgeleitet.

Bevorzugt wird **S_{RDX2}** unterhalb des Brüdenstroms **S_{RDB2}** an **RD₂** entnommen. Insbesondere ist **S_{RDX2}** dann ausgewählt aus Sumpfstrom **S_{RDX2S}**, Zwischenstrom **S_{RDX2Z}**.

Ein Sumpfstrom **S_{RDX2S}** ist ein Strom, dessen Entnahmestelle an **RD₂** auf gleicher Höhe wie die Entnahmestelle von **S_{RDS2}** oder darunter liegt. **S_{RDX2S}** kann dann durch einen Wärmeüberträger **WT,** insbesondere einen Sumpfverdampfer **VS** geleitet werden, und in diesem Energie von **S_{RDB1}** auf **S_{RDX2S}** übertragen werden.

Ein Zwischenstrom **S_{RDX2Z}** ist ein Strom, dessen Entnahmestelle an **RD₂** zwischen den Entnahmestellen von **S_{RDB2}** und **S_{RDS2}** liegt. **S_{RDX2Z}** kann dann aus **RD₂** und durch einen Wärmeüberträger **WT,** insbesondere einen Zwischenverdampfer **VZ** geleitet werden, und in diesem Energie von **S_{RDB1}** auf **S_{RDX2Z}** übertragen werden.

(β-iii) Energie wird von **S_{RDB1}** auf mindestens einen von **G_{RD2}** verschiedenen Wärmeträger **Wᵢᵢᵢ₁** übertragen, und der mindestens eine Wärmeträger **Wᵢᵢᵢ₁** wird dann durch **RD₂** geleitet, wodurch Energie von dem mindestens eine Wärmeträger **Wᵢᵢᵢ₁** auf **G_{RD2}** übertragen wird.

Eine solche Ausführungsform kann beispielsweise realisiert werden, in dem der mindestens eine Wärmeträger **Wᵢᵢᵢ₁** durch die Rektifikationskolonne **RD₂** durch eine Leitung geführt wird, über deren Oberfläche der mindestens eine Wärmeträger **Wᵢᵢᵢ₁** Energie auf **G_{RD2}** in **RD₂** überträgt.

Als Wärmeträger **W₁ W₂, W₃, W₄, W₅** bzw. mindestens einen Wärmeträger **Wᵢ₁** bzw. mindestens einen Wärmeträger **Wᵢᵢ₁** bzw. mindestens einen Wärmeträger **Wᵢᵢᵢ₁** kann jeder dem Fachmann bekannte Wärmeträger genutzt werden. Bevorzugt sind solche Wärmeträger ausgewählt aus der Gruppe bestehend aus Wasser; Alkohol-Wasser-Lösungen; Salz-Wasser-Lösungen, wozu auch ionische Flüssigkeiten, wie zum Beispiel LiBr-Lösungen, Dialkylimidazoliumsalze wie insbesondere Dialkylimidazoliumdialkylphosphate, gehören; Mineralöle, wie zum Beispiel Dieselöle; Thermalöle wie zum Beispiel Silikonöle; biologische Öle wie zum Beispiel Limonen; aromatische Kohlenwasserstoffe wie zum Beispiel Dibenzyltoluol. Bevorzugtester Wärmeträger ist Wasser.

Salz-Wasser-Lösungen, die verwendet werden können, sind beispielsweise auch in der DE 10 2005 028 451 A1 und der WO 2006/134015 A1 beschrieben.

### 4.9 Zugabe von Frischalkohol

Im erfindungsgemäßen Verfahren wird der Alkohol ROH verbraucht, und insbesondere bei kontinuierlicher Verfahrensführung muss dieser deshalb mit frischem Alkohol ROH ersetzt werden.

Frischer Alkohol wird insbesondere in mindestens eine der Kolonnen ausgewählt aus Rektifikationskolonne **RD₁,** Rektifikationskolonne **RD₂,** Reaktivrektifikationskolonne **RR_{A}** zugegeben und, wenn Schritt (a2) durchgeführt wird, alternativ oder zusätzlich in die Reaktivrektifikationskolonne **RR_{B}** zugegeben.

In einer bevorzugten Ausführungsform der vorliegenden Erfindung wird demnach ein von **S_{AE1}** und **S_{BE1}** verschiedener Strom **S_{XE1}** umfassend ROH in mindestens eine der Kolonnen ausgewählt aus Rektifikationskolonne **RD₁,** Rektifikationskolonne **RD₂,** Reaktivrektifikationskolonne **RR_{A}** zugegeben und, wenn Schritt (a2) durchgeführt wird, alternativ oder zusätzlich in die Reaktivrektifikationskolonne **RR_{B}** zugegeben.

Die Zuführung des frischen Alkohols ROH erfolgt dabei insbesondere direkt als Eduktstrom **S_{AE1}** umfassend ROH in die Reaktionskolonne **RR_{A}** bzw. in den Ausführungsformen, in denen Schritt (a2) durchgeführt wird, in die Reaktionskolonnen **RR_{A}** und **RR_{B}.**

Im erfindungsgemäßen Verfahren ist es weiterhin bevorzugt, den ROH umfassende Brüdenstrom **S_{RDB1}** mindestens teilweise als Eduktstrom **S_{AE1}** in Schritt (a1) und gegebenenfalls als Eduktstrom **S_{BE1}** in Schritt (a2) einzusetzen. Alternativ oder zusätzlich kann der Brüdenstrom **S_{RDB2}** mindestens teilweise als Eduktstrom **S_{AE1}** in Schritt (a1) und gegebenenfalls als Eduktstrom **S_{BE1}** in Schritt (a2) eingesetzt werden.

In der besonders bevorzugten Ausführungsform, in der **S_{RDB1}** und **S_{RDB2}** mindestens teilweise als Eduktstrom **S_{AE1}** in Schritt (a1) und gegebenenfalls als Eduktstrom **S_{BE1}** in Schritt (a2) eingesetzt werden, können **S_{RDB1}** und **S_{RDB2}** getrennt voneinander der jeweiligen Reaktivrektifikationskolonne **RR_{A}** bzw. **RR_{B}** zugeführt werden oder erst miteinander vermischt und dann der jeweiligen Reaktivrektifikationskolonne **RR_{A}** bzw. **RR_{B}** zugeführt werden. Bevorzugt werden **S_{RDB1}** und **S_{RDB2}** erst miteinander vermischt und dann der jeweiligen Reaktivrektifikationskolonne **RR_{A}** bzw. **RR_{B}** zugeführt.

In dieser bevorzugten Ausführungsform ist es noch bevorzugter, wenn der frische Alkohol ROH einer der Rektifikationskolonnen **RD₁, RD₂,** bevorzugt **RD₁,** zugegeben wird.

Wenn der frische Alkohol ROH der Rektifikationskolonne **RD₁** oder **RD₂** zugegeben wird, wird er bevorzugt entweder im Verstärkungsteil der jeweiligen Rektifikationskolonne oder direkt am Kopf der jeweiligen Rektifikationskolonne zugeführt. Die optimale Zulaufstelle ist abhängig vom Wassergehalt des eingesetzten frischen Alkohols und andererseits von dem gewünschten Restwassergehalt im Brüdenstrom **S_{RDB1}** bzw. **S_{RDB2}.** Je höher der Wasseranteil im eingesetzten Alkohol und je höher die Reinheitsanforderung im Brüdenstrom **S_{RDB1}** bzw. **S_{RDB2}** ist, um so günstiger ist ein Zulauf einige theoretische Böden unterhalb des Kopfes der Rektifikationskolonne **RD₁** bzw. **RD₂.** Bevorzugt sind bis zu 20 theoretische Böden unterhalb des Kopfes der Rektifikationskolonne **RD₁** bzw. **RD₂** und insbesondere 1 bis 5 theoretische Böden.

Wenn der frische Alkohol ROH der Rektifikationskolonne **RD₁** bzw. **RD₂** zugegeben wird, wird er bei Temperaturen bis hin zum Siedepunkt, bevorzugt bei Raumtemperatur, am Kopf der Rektifikationskolonne **RD₁** bzw. **RD₂** zugegeben. Hierbei kann für den frischen Alkohol ein eigener Zulauf vorgesehen sein oder aber bei Rückführung eines Teils des am Kopf der Rektifikationskolonne **RD₁** bzw. **RD₂** entnommenen Alkohols nach der Kondensation mit diesem gemischt und gemeinsam in die Rektifikationskolonne **RD₁** bzw. **RD₂** zugeführt werden. In diesem Fall ist es besonders bevorzugt, wenn der frische Alkohol in einen Kondensatbehälter, in dem der aus dem Brüdenstrom **S_{RDB1}** bzw. **S_{RDB2}** kondensierte Alkohol gesammelt wird, zugegeben wird.

### 5. Beispiele

### 5.1 Beispiel 1 (erfindungsgemäß)

Der Aufbau gemäß Beispiel 1 entspricht der Zweikolonnenverschaltung nach Abbildung 1, wobei **p₁ > p_{3A} > p₂.**

Ein Strom **S_{AE2}** <3A02> wässriger NaOH (50 Gew.-%) von 5 t/h wird mit 25 °C am Kopf einer Reaktionskolonne **RR_{A}** <3A> zugeführt. Im Gegenstrom wird ein dampfförmiger Methanolstrom **S_{AE1}** <3A01> von 70.2 t/h über dem Sumpf der Reaktionskolonne **RR_{A}** <3A> zugeführt. Die Reaktionskolonne **RR_{A}** <3A> wird bei einem Druck **p_{3A}** von 1.6 bar betrieben. Am Sumpf der Kolonne **RR_{A}** <3A> wird ein nahezu wasserfreier Produktstrom **S_{AP*}** <3A08> von 10.8 t/h entnommen (30 Gew.-% Natriummethanolat in Methanol). Am Verdampfer **VS_{3A}** <3A06> der Reaktionskolonne **RR_{A}** <3A> werden ca. 0.7 MW Heizleistung mit Hilfe von Heizdampf eingespeist. Ein dampfförmiger Methanol-Wasser-Strom **S_{AB}** <3A03> wird am Kopf der Reaktionskolonne **RR_{A}** <3A> entnommen. Ein Teil dieses Stromes wird über einen Kondensator **K_{RRA}** <3A05> auf die Reaktionskolonne **RR_{A}** <3A> rückgeführt, und der übrige Teil (64.4 t/h) in einem Verdichter **VD₃₁** <10> auf 7.1 bar verdichtet, wobei ca. 4 MW Verdichterleistung notwendig sind, und einer ersten Rektifikationskolonne **RD₁** <1> zugeführt. Die Rektifikationskolonne **RD₁** <1> wird bei **p₁** = ~ 7 bar betrieben. Am Kopf der Rektifikationskolonne **RD₁** <1> wird ein flüssiger Frisch-Methanol-Strom von 9.5 t/h zugeführt (nicht in Abbildung 1 gezeigt) und dampfförmiger Methanolstrom **S_{RDB1}** <101> entnommen. Ein Teil von **S_{RDB1}** <101> wird über den Kondensator **K_{RD1}** <102> in Kolonne **RD₁** <1> rückgeführt. Der übrige Teil von **S_{RDB1}** <101> (42.9 t/h) wird der Reaktionskolonne **RR_{A}** <3A> zugeführt. Im Kondensator **K_{RD1}** <102> von Kolonne **RD₁** <1>, der zugleich der Verdampfer **VS_{RD2}** der zweiten Rektifikationskolonne **RD₂** <2> ist, wird die Heizleistung für die Kolonne **RD₂** <2> zur Verfügung gestellt. In der Ausführungsform gemäß Beispiel 1 wurde die direkte Kontaktierung genutzt, bei der der Kondensator **K_{RD1}** <102> gleichzeitig als Sumpfverdampfer **VS_{RD2}** <204> eingesetzt wird.

Am Sumpf der Rektifikationskolonne **RD₁** <1>, wird ein flüssiger Strom eines Wasser-Methanol-Gemisches **S_{RDS1}** <103> abgeführt, von dem ein Teil **S_{RDS12}** <104> von 30.9 t/h in die Rektifikationskolonne **RD₂** <2> geleitet wird, und der restliche Teil des Stromes **S_{RDS1}** <103> als **S_{RDS11}** <105> in **RD₁** <1> rückgeführt wird. Am Verdampfer **VS_{RD1}** <106> der Rektifikationskolonne **RD₁** <1> werden ca. 5.4 MW Heizleistung über Heizdampf eingespeist.

Die Rektifikationskolonne **RD₂** <2> wird bei einem Druck **p₂** von 1.1 bar betrieben. Am Kopf der Rektifikationskolonne **RD₂** <2> wird ein dampfförmiger Methanolstrom **S_{RDB2}** <201> entnommen. Ein Teil von **S_{RDB2}** <201> wird über den Kondensator **K_{RD2}** <203> in Kolonne **RD₂** <2> rückgeführt. Der übrige Teil von **S_{RDB2}** <201> (27.3 t/h) wird der Reaktionskolonne **RR_{A}** <3A> zugeführt. Dieser Teil des dampfförmigen Stroms **S_{RDB2}** <201> wird in einem Verdichter **VD₂₃** <13> auf 2 bar verdichtet, wobei ca. 0.6 MW Verdichterleistung notwendig sind. Am Sumpf der Rektifikationskolonne **RD₂** <2> wird ein flüssiger Strom Wasser **S_{RDS2}** <202> (verunreinigt mit 500 Gew.-ppm Methanol) von 3.7 t/h abgeführt. Zur Verdampfung an der Rektifikationskolonne **RD₂** <2> (da direkte Wärmeintegration erfolgt, wird die in Abbildung 1 gezeigte Funktion des Sumpfverdampfers **VS_{RD2}** <204> durch den Kondensator **K_{RD1}** <102> mitübernommen) werden ca. 14.8 MW Heizleistung über Wärmeintegration mit der Kolonne **RD₁** <1> auf einen Teil **S_{RDS22}** <222> von **S_{RDS2}** <202> eingespeist.

Die jeweiligen, nicht rückgeführten Teile der an den Köpfen von **RD₁** <1> und **RD₂** <2> entnommenen dampfförmigen Methanolströme **S_{RDB1}** <101> und **S_{RDB2}** <201> werden gemischt und wieder der Reaktionskolonne **RR_{A}** <3A> am Sumpf zugeführt.

In Summe werden in diesem Beispiel ca. 6.1 MW Heizleistung über Heizdampf und ca. 4.6 MW elektrische Leistung (Verdichterleistung) benötigt und müssen extern bereitgestellt werden.

### 5.2 Beispiel 2 (erfindungsgemäß)

Der Aufbau gemäß Beispiel 2 entspricht der Zweikolonnenverschaltung nach Abbildung 2, wobei **p₁** > **p₂** > **p_{3A}.**

Ein Strom **S_{AE2}** <3A02> wässriger NaOH (50 Gew.-%) von 5 t/h wird mit 25 °C am Kopf einer Reaktionskolonne **RR_{A}** <3A> zugeführt. Im Gegenstrom wird ein dampfförmiger Methanolstrom **S_{AE1}** <3A01> von 70.2 t/h über dem Sumpf der Reaktionskolonne **RR_{A}** <3A> zugeführt.

Die Reaktionskolonne **RR_{A}** <3A> wird bei einem Druck **p_{3A}** von 1.1 bar betrieben. Am Sumpf der Kolonne **RR_{A}** <3A> wird ein nahezu wasserfreier Produktstrom **S_{AP*}** <3A08> von 10.8 t/h entnommen (30 Gew.-% Natriummethanolat in Methanol). Am Verdampfer **VS_{3A}** <3A06> der Reaktionskolonne **RR_{A}** <3A> werden ca. 2.4 MW Heizleistung mit Hilfe von Heizdampf eingespeist. Ein dampfförmiger Methanol-Wasser-Strom **S_{AB}** <3A03> wird am Kopf der Reaktionskolonne **RR_{A}** <3A> entnommen. Ein Teil dieses Stromes wird über einen Kondensator **K_{RRA}** <3A05> auf die Reaktionskolonne **RR_{A}** <3A> rückgeführt, und der übrige Teil (64.4 t/h) in einem Verdichter **VD₃₁** <10> auf 9 bar verdichtet, wobei ca. 5.8 MW Verdichterleistung notwendig sind, und einer ersten Rektifikationskolonne **RD₁** <1> zugeführt. Die Rektifikationskolonne **RD₁** <1> wird bei **p₁** = ~ 8.9 bar betrieben. Am Kopf der Rektifikationskolonne **RD₁** <1> wird ein flüssiger Frisch-Methanol-Strom von 9.5 t/h zugeführt (nicht in Abbildung 2 gezeigt) und dampfförmiger Methanolstrom **S_{RDB1}** <101> entnommen. Ein Teil von **S_{RDB1}** <101> wird über den Kondensator **K_{RD1}** <102> in Kolonne **RD₁** <1> rückgeführt. Der übrige Teil von **S_{RDB1}** <101> (42.9 t/h) wird der Reaktionskolonne **RR_{A}** <3A> zugeführt. Im Kondensator **K_{RD1}** <102> von Kolonne **RD₁** <1>, der zugleich der Verdampfer **VS_{RD2}** <204> der zweiten Rektifikationskolonne **RD₂** <2> ist, wird die Heizleistung für die Kolonne **RD₂** <2> zur Verfügung gestellt. In der Ausführungsform gemäß Beispiel 2 wurde die direkte Kontaktierung genutzt, bei der der Kondensator **K_{RD1}** <102> gleichzeitig als Sumpfverdampfer **VS_{RD2}** <204> eingesetzt wird.

Am Sumpf der Rektifikationskolonne **RD₁** <1> wird ein flüssiger Strom eines Wasser-Methanol-Gemisches **S_{RDS1}** <103> abgeführt, von dem ein Teil **S_{RDS12}** <104> von 31.9 t/h in die Rektifikationskolonne **RD₂** <2> geleitet wird, und der restliche Teil des Stromes **S_{RDS1}** <103> als **S_{RDS11}** <105> in **RD₁** <1> rückgeführt wird. Am Verdampfer **VS_{RD1}** <106> der Rektifikationskolonne **RD₁** <1> werden ca. 5.2 MW Heizleistung über Heizdampf eingespeist.

Die Rektifikationskolonne **RD₂** <2> wird bei einem Druck **p₂** von 1.5 bar betrieben. Am Kopf der Rektifikationskolonne **RD₂** <2> wird ein dampfförmiger Methanolstrom **S_{RDB2}** <201> entnommen. Ein Teil von **S_{RDB2}** <201> wird über den Kondensator **K_{RD2}** <203> in Kolonne **RD₂** <2> rückgeführt. Der übrige Teil von **S_{RDB2}** <201> (28.2 t/h) wird der Reaktionskolonne **RR_{A}** <3A> zugeführt. Am Sumpf der Rektifikationskolonne **RD₂** <2> wird ein flüssiger Strom Wasser **S_{RDS2}** <202> (verunreinigt mit 500 Gew.-ppm Methanol) von 3.7 t/h abgeführt. Zur Verdampfung an der Rektifikationskolonne **RD₂** <2> (da direkte Wärmeintegration erfolgt, wird die in Abbildung 1 gezeigte Funktion des Sumpfverdampfers **VS_{RD2}** <204> durch den Kondensator **K_{RD1}** <102> mitübernommen) werden ca. 15.9 MW Heizleistung über Wärmeintegration mit der Kolonne **RD₁** <1> > auf einen Teil **S_{RDS22}** <222> von **S_{RDS2}** <202> eingespeist.

Die jeweiligen, nicht rückgeführten Teile der an den Köpfen von **RD₁** <1> und **RD₂** <2> entnommenen dampfförmigen Methanolströme **S_{RDB1}** <101> und **S_{RDB2}** <201> werden gemischt und wieder der Reaktionskolonne **RR_{A}** <3A> am Sumpf zugeführt.

In Summe werden in diesem Beispiel ca. 7.6 MW Heizleistung über Heizdampf und ca. 5.8 MW elektrische Leistung (Verdichterleistung) benötigt und müssen extern bereitgestellt werden.

### 5.3 Beispiel 3 (nicht erfindungsgemäß)

Der Aufbau gemäß Beispiel 3 entspricht der Zweikolonnenverschaltung nach Abbildung 3, wobei **p₂** > **p₁** > **p_{3A}.** Der in Abbildung 3 gezeigte Zwischenverdampfer **VZ_{RD1}** <107> mit dem an der Entnahmestelle <111> entnommenen Strom **S_{RDX1}** <112> wird im Aufbau gemäß Beispiel 3 ebenfalls weggelassen. Der Kondensator **K_{RD2}** <203> ist außerdem zugleich der Sumpfverdampfer **VS_{RD1}** <106>. Der Frisch-Methanol-Strom **S_{XE1}** <205> wird in Abbildung 3 an der Rektifikationskolonne **RD₂** <2> zugeführt, im Aufbau nach Beispiel 3 aber an **RD₁** <1>

Ein Strom **S_{AE2}** <3A02> wässriger NaOH (50 Gew.-%) von 5 t/h wird mit 25 °C am Kopf einer Reaktionskolonne **RR_{A}** <3A> zugeführt. Im Gegenstrom wird ein dampfförmiger Methanolstrom **S_{AE1}** <3A01> von 70.2 t/h über dem Sumpf der Reaktionskolonne **RR_{A}** <3A> zugeführt. Die Reaktionskolonne **RR_{A}** <3A> wird bei einem Druck **p_{3A}** von 1.1 bar betrieben. Am Sumpf der Kolonne **RR_{A}** <3A> wird ein nahezu wasserfreier Produktstrom **S_{AP*}** <3A08> von 10.8 t/h entnommen (30 Gew.-% Natriummethanolat in Methanol). Am Verdampfer **VS_{3A}** <3A06> der Reaktionskolonne **RR_{A}** <3A> werden ca. 1.4 MW Heizleistung mit Hilfe von Heizdampf eingespeist.

Ein dampfförmiger Methanol-Wasser-Strom **S_{AB}** <3A03> wird am Kopf der Reaktionskolonne **RR_{A}** <3A> entnommen. Ein Teil dieses Stromes wird über einen Kondensator **K_{RRA}** <3A05> auf die Reaktionskolonne **RR_{A}** <3A> rückgeführt, und der übrige Teil (64.4 t/h) in einem Verdichter **VD₃₁** <10> auf 1.7 bar verdichtet, wobei ca. 1.1 MW Verdichterleistung notwendig sind, und einer ersten Rektifikationskolonne **RD₁** <1> zugeführt. Die Rektifikationskolonne **RD₁** <1> wird bei **p₁** = ~ 1.5 bar betrieben. Am Kopf der Rektifikationskolonne **RD₁** <1> wird ein flüssiger Frisch-Methanol-Strom **S_{XE1}** <205> von 9.5 t/h zugeführt (in Abbildung 3 am Kopf der Rektifikationskolonne **RD₂** <2> gezeigt) und dampfförmiger Methanolstrom **S_{RDB1}** <101> entnommen. Ein Teil von **S_{RDB1}** <101> wird über den Kondensator **K_{RD1}** <102> in Kolonne **RD₁** <1> rückgeführt. Der übrige Teil von **S_{RDB1}** <101> (56.8 t/h) wird der Reaktionskolonne **RR_{A}** <3A> zugeführt. In der Ausführungsform gemäß Beispiel 3 wurde die direkte Kontaktierung genutzt, bei der der Kondensator **K_{RD2}** <203> gleichzeitig als Sumpfverdampfer **VS_{RD1}** <106> dient.

Am Sumpf der Rektifikationskolonne **RD₁** <1> wird ein flüssiger Strom eines Wasser-Methanol-Gemisches **S_{RDS1}** <103> abgeführt, von dem ein Teil **S_{RDS12}** <104> von 17 t/h in die Rektifikationskolonne **RD₂** <2> geleitet wird, und der restliche Teil des Stromes **S_{RDS1}** <103> als **S_{RDS11}** <105> in **RD₁** <1> rückgeführt wird.

Der Druck des abgeführten Stroms **S_{RDS12}** <104> wird in einer Pumpe P <15> auf 9 bar erhöht und der Strom **S_{RDS12}** <104> der zweiten Rektifikationskolonne **RD₂** <2> zugeführt. Die Rektifikationskolonne **RD₂** <2> wird bei einem Druck **p₂** von 8.9 bar betrieben. Im Kondensator **K_{RD2}** <203> von Kolonne **RD₂**<2>, der zugleich der Verdampfer der Kolonne **RD₁** <1> ist, werden ca. 8.2 MW an Heizleistung für die Kolonne **RD₁** <1> zur Verfügung gestellt. Am Kopf der Rektifikationskolonne **RD₂**<2> wird ein dampfförmiger Methanolstrom **S_{RDB2}** <201> entnommen. Ein Teil von **S_{RDB2}** <201> wird über den Kondensator **K_{RD2}** <203> in Kolonne **RD₂** <2> rückgeführt. Der übrige Teil von **S_{RDB2}** <201> (13.4 t/h) wird der Reaktionskolonne **RR_{A}** <3A> zugeführt. Am Sumpf der Rektifikationskolonne **RD₂** <2> wird ein flüssiger Strom Wasser (verunreinigt mit 500 Gew.-ppm Methanol) von 3.7 t/h abgeführt. Am Verdampfer **VS_{RD2}** <204> der Rektifikationskolonne **RD₂** <2> werden ca. 12.9 MW Heizleistung mit Hilfe von Heizdampf eingespeist.

Die jeweiligen, nicht rückgeführten Teile der an den Köpfen von **RD₁** <1> und **RD₂** <2> entnommenen dampfförmigen Methanolströme **S_{RDB1}** <101> und **S_{RDB2}** <201> werden gemischt, entspannt und wieder der Reaktionskolonne **RR_{A}** <3A> am Sumpf zugeführt.

In Summe werden in diesem Beispiel ca. 14.3 MW Heizleistung über Heizdampf und ca. 1.1 MW elektrische Leistung (Verdichterleistung) benötigt und müssen extern bereitgestellt werden.

### 5.4 Beispiel 4 (nicht erfindungsgemäß)

Der Aufbau gemäß Beispiel 4 entspricht der Zweikolonnenverschaltung nach Abbildung 4, wobei **p₂ > p_{3A} > p₁.** Der in Abbildung 4 gezeigte Zwischenverdampfer **VZ_{RD1}** <107> mit dem an der Entnahmestelle <111> entnommenen Strom **S_{RDX1}** <112> wird im Aufbau gemäß Beispiel 4 ebenfalls weggelassen. Der Kondensator **K_{RD2}** <203> ist außerdem zugleich der Sumpfverdampfer **VS_{RD1}** <106>. Der Frisch-Methanol-Strom **S_{XE1}** <205> wird in Abbildung 4 an der Rektifikationskolonne **RD₂** <2> zugeführt, im Aufbau nach Beispiel 4 aber an **RD₁** <1>

Ein Strom **S_{AE2}** <3A02> wässriger NaOH (50 Gew.-%) von 5 t/h wird mit 25 °C am Kopf einer Reaktionskolonne **RR_{A}** <3A> zugeführt. Im Gegenstrom wird ein dampfförmiger Methanolstrom **S_{AE1}** <3A01> von 70.2 t/h über dem Sumpf der Reaktionskolonne **RR_{A}** <3A> zugeführt. Die Reaktionskolonne **RR_{A}** <3A> wird bei einem Druck **p_{3A}** von 1.6 bar betrieben. Am Sumpf der Kolonne **RR_{A}** <3A> wird ein nahezu wasserfreier Produktstrom **S_{AP*}** <3A08> von 10.8 t/h entnommen (30 Gew.-% Natriummethanolat in Methanol). Am Verdampfer **VS_{3A}** <3A06> der Reaktionskolonne **RR_{A}** <3A> werden ca. 0.8 MW Heizleistung mit Hilfe von Heizdampf eingespeist. Ein dampfförmiger Methanol-Wasser-Strom **S_{AB}** <3A03> wird am Kopf der Reaktionskolonne **RR_{A}** <3A> entnommen. Ein Teil dieses Stromes wird über einen Kondensator **K_{RRA}** <3A05> auf die Reaktionskolonne **RR_{A}** <3A> rückgeführt, und der übrige Teil (64.4 t/h) einer ersten Rektifikationskolonne **RD₁** <1> zugeführt. Die Rektifikationskolonne **RD₁** <1> wird bei **p₁** = ~ 1.1 bar betrieben. Am Kopf der Rektifikationskolonne **RD₁** <1> wird ein flüssiger Frisch-Methanol-Strom **S_{XE1}** <205> von 9.5 t/h zugeführt (in Abbildung 4 am Kopf der Rektifikationskolonne **RD₂** <2> gezeigt) und dampfförmiger Methanolstrom **S_{RDB1}** <101> entnommen.

Ein Teil von **S_{RDB1}** <101> wird über den Kondensator **K_{RD1}** <102> in Kolonne **RD₁** <1> rückgeführt. Der übrige Teil von **S_{RDB1}** <101> (55 t/h) wird in einem Verdichter **VD₁₃** <16> auf 2 bar verdichtet, wobei ca. 1.2 MW Verdichterleistung notwendig sind, und der Reaktionskolonne **RR_{A}** <3A> zugeführt. Am Sumpf der Rektifikationskolonne **RD₁** <1> wird ein flüssiger Strom eines Wasser-Methanol-Gemisches **S_{RDS1}** <103> abgeführt, von dem ein Teil **S_{RDS12}** <104> von 18.9 t/h in die Rektifikationskolonne **RD₂** <2> geleitet wird, und der restliche Teil des Stromes **S_{RDS1}** <103> als **S_{RDS11}** <105> in **RD₁** <1> rückgeführt wird.

Der Druck des abgeführten Stroms **S_{RDS12}** <104> wird in einer Pumpe **P** <15> auf 3.4 bar erhöht und der Strom der zweiten Rektifikationskolonne **RD₂** <2> zugeführt. Die Rektifikationskolonne **RD₂** <2> wird bei einem Druck **p₂** von 3.2 bar betrieben. Im Kondensator **K_{RD2}** <203> von Kolonne **RD₂**<2>, der zugleich der Verdampfer der Kolonne **RD₁** <1> ist, werden ca. 6.3 MW an Heizleistung für die Kolonne **RD₁** <1> zur Verfügung gestellt. Am Kopf der Rektifikationskolonne **RD₂** <2> wird ein dampfförmiger Methanolstrom **S_{RDB2}** <201> entnommen. Ein Teil von **S_{RDB2}** <201> wird über den Kondensator **K_{RD2}** <203> in Kolonne **RD₂** <2> rückgeführt. Der übrige Teil von **S_{RDB2}** <201> (15.2 t/h) wird der Reaktionskolonne **RR_{A}** <3A> zugeführt. Am Sumpf der Rektifikationskolonne **RD₂** <2> wird ein flüssiger Strom Wasser (verunreinigt mit 500 Gew.-ppm Methanol) von 3.7 t/h abgeführt. Am Verdampfer **VS_{RD2}** <204> der Rektifikationskolonne **RD₂** <2> werden ca. 11.4 MW Heizleistung mit Hilfe von Heizdampf eingespeist.

Die jeweiligen, nicht rückgeführten Teile der an den Köpfen von **RD₁** <1> und **RD₂** <2> entnommenen dampfförmigen Methanolströme **S_{RDB1}** <101> und **S_{RDB2}** <201> werden gemischt und wieder der Reaktionskolonne **RR_{A}** <3A> am Sumpf zugeführt.

In Summe werden in diesem Beispiel ca. 12.2 MW Heizleistung über Heizdampf und ca. 1.2 MW elektrische Leistung (Verdichterleistung) benötigt und müssen extern bereitgestellt werden.

### 5.5 Ergebnis

Aus dem Vergleich des Anteils an Heizdampf und elektrischem Strom, der zur Deckung des Energiebedarfs bei den erfindungsgemäßen und nicht erfindungsgemäßen Beispielen nötig ist, ist ersichtlich, dass das erfindungsgemäße Verfahren es überraschend erlaubt, einen großen Teil des Energiebedarfs durch elektrische Energie zu decken, und den Anteil der durch Heizdampf zu erbringenden Leistung zu minimieren.

## Patentansprüche

1. Verfahren zur Herstellung mindestens eines Alkalimetallalkoholats der Formel M_{A}OR, wobei R ein C₁ bis C₆-Kohlenwasserstoffrest ist, und wobei M_{A} ausgewählt aus Natrium, Kalium ist, wobei:
(a1) ein Eduktstrom **S_{AE1}** umfassend ROH mit einem Eduktstrom **S_{AE2}** umfassend M_{A}OH im Gegenstrom bei einem Druck **p_{3A}** und einer Temperatur **T_{3A}** in einer Reaktivrektifikationskolonne **RR_{A}** zu einem Rohprodukt **RP_{A}** umfassend M_{A}OR, Wasser, ROH, M_{A}OH umgesetzt wird,
wobei am unteren Ende von **RR_{A}** ein Sumpfproduktstrom **S_{AP}** umfassend ROH und M_{A}OR entnommen wird und am oberen Ende von **RR_{A}** ein Brüdenstrom **S_{AB}** umfassend Wasser und ROH entnommen wird,
(a2) und gegebenenfalls, gleichzeitig mit und räumlich getrennt von Schritt (a1), ein Eduktstrom **S_{BE1}** umfassend ROH mit einem Eduktstrom **S_{BE2}** umfassend M_{B}OH im Gegenstrom bei einem Druck **p_{3B}** und einer Temperatur **T_{3B}** in einer Reaktivrektifikationskolonne **RR_{B}** zu einem Rohprodukt **RP_{B}** umfassend M_{B}OR, Wasser, ROH, M_{B}OH umgesetzt wird, wobei M_{B} ausgewählt aus Natrium, Kalium ist,
wobei am unteren Ende von **RR_{B}** ein Sumpfproduktstrom **S_{BP}** umfassend ROH und M_{B}OR entnommen wird und am oberen Ende von **RR_{B}** ein Brüdenstrom **S_{BB}** umfassend Wasser und ROH entnommen wird,
(b) der Brüdenstrom **S_{AB},** und, wenn Schritt (a2) durchgeführt wird, der Brüdenstrom **S_{BB},** vermischt mit **S_{AB}** oder getrennt von **S_{AB},** in eine erste Rektifikationskolonne **RD₁** geleitet wird,
wodurch ein Gemisch **G_{RD1}** umfassend Wasser und ROH in der ersten Rektifikationskolonne **RD₁** erhalten wird,
(c) das Gemisch **G_{RD1}** in der ersten Rektifikationskolonne **RD₁** bei einem Druck **p₁** und einer Temperatur **T₁** in einen ROH umfassenden Brüdenstrom **S_{RDB1}** am oberen Ende von **RD₁** und einen Sumpfstrom **S_{RDS1}** umfassend Wasser und ROH am unteren Ende von **RD₁** aufgetrennt wird,
(d) der Sumpfstrom **S_{RDS1}** ganz oder teilweise in eine zweite Rektifikationskolonne **RD₂** geleitet wird,
wodurch ein Gemisch **G_{RD2}** umfassend Wasser und ROH in der zweiten Rektifikationskolonne **RD₂** erhalten wird,
(e) das Gemisch **G_{RD2}** bei einem Druck **p₂** und einer Temperatur **T₂** in einen ROH umfassenden Brüdenstrom **S_{RDB2}** am Kopf von **RD₂** und einen Sumpfstrom **S_{RDS2}** umfassend Wasser am unteren Ende von **RD₂** aufgetrennt wird,
**dadurch gekennzeichnet,**
**dass p₁** > **p₂, p₁** > **p_{3A}** gilt, und in den Fällen, in denen Schritt (a2) durchgeführt wird, **p₁** > **p_{3B}** gilt,
und **dass** (f) Energie von **S_{RDB1}** auf das Gemisch **G_{RD2}** in der zweiten Rektifikationskolonne **RD₂** übertragen wird.

2. Verfahren nach Anspruch 1, wobei in Schritt (f) Energie von **S_{RDB1}** auf **G_{RD2}** direkt übertragen wird.

3. Verfahren nach Anspruch 2, wobei mindestens einer der Schritte (α-i), (α-ii), (α-iii) durchgeführt wird:
(α-i) Energie von **S_{RDB1}** wird auf einen Teil **S_{RDS22}** des aus **RD₂** geleiteten Sumpfstroms **S_{RDS2}** übertragen und **S_{RDS22}** dann in **RD₂** zurückgeleitet;
(α-ii) mindestens ein von **S_{RDB2}** und **S_{RDS2}** verschiedener Strom **S_{RDX2}** umfassend ROH und Wasser wird aus **RD₂** geleitet, dann wird Energie von **S_{RDB1}** auf **S_{RDX2}** übertragen und **S_{RDX2}** in **RD₂** zurückgeleitet;
(α-iii) **S_{RDB1}** wird durch **RD₂** geleitet, wodurch Energie von **S_{RDB1}** auf **G_{RD2}** übertragen wird.

4. Verfahren nach Anspruch 1, wobei in Schritt (f) Energie von **S_{RDB1}** auf **G_{RD2}** indirekt übertragen wird.

5. Verfahren nach Anspruch 4, wobei mindestens einer der Schritte (β-i), (β-ii), (β-iii) durchgeführt wird:
(β-i) ein Teil **S_{RDS22}** des aus **RD₂** geleiteten Sumpfstroms **S_{RDS2}** wird in die zweite Rektifikationskolonne **RD₂** zurückgeleitet, wobei Energie von **S_{RDB1}** auf mindestens einen von **S_{RDS22}** verschiedenen Wärmeträger **Wᵢ₁** übertragen und dann von dem mindestens einen Wärmeträger **Wᵢ₁** auf **S_{RDS22}** übertragen wird, und **S_{RDS22}** dann in **RD₂** zurückgeleitet wird;
(β-ii) mindestens ein von **S_{RDB2}** und **S_{RDS2}** verschiedener Strom **S_{RDX2}** umfassend ROH und Wasser wird aus **RD₂** geleitet, und Energie wird von **S_{RDB1}** auf mindestens einen von **S_{RDX2}** verschiedenen Wärmeträger **Wᵢᵢ₁** übertragen und dann von dem mindestens einen Wärmeträger **Wᵢᵢ₁** auf **S_{RDX2}** übertragen, und dann wird **S_{RDX2}** in **RD₂** zurückgeleitet;
(β-iii) Energie wird von **S_{RDB1}** auf mindestens einen von **G_{RD2}** verschiedenen Wärmeträger **Wᵢᵢᵢ₁** übertragen, und der mindestens eine Wärmeträger **Wᵢᵢᵢ₁** wird dann durch **RD₂** geleitet, wodurch Energie von dem mindestens eine Wärmeträger **Wᵢᵢᵢ₁** auf **G_{RD2}** übertragen wird.

6. Verfahren nach Anspruch 5, wobei **Wᵢ₁, Wᵢᵢ₁**, **Wᵢᵢᵢ₁** jeweils Wasser ist.

7. Verfahren nach einem der Ansprüche 3, 5 und 6, wobei **S_{RDX2}** unterhalb des Brüdenstrom **S_{RDB2}** an **RD₂** entnommen wird.

8. Verfahren nach einem der Ansprüche 1 bis 7, wobei **S_{RDB2}** mindestens teilweise als Eduktstrom **S_{AE1}** in der Reaktivrektifikationskolonne **RR_{A}** und, wenn Schritt (a2) durchgeführt wird, alternativ oder zusätzlich als Eduktstrom **S_{BE1}** in der Reaktivrektifikationskolonne **RR_{B}** eingesetzt wird.

9. Verfahren nach einem der Ansprüche 1 bis 8, wobei **S_{RDB1}** mindestens teilweise als Eduktstrom **S_{AE1}** in der Reaktivrektifikationskolonne **RR_{A}** und, wenn Schritt (a2) durchgeführt wird, alternativ oder zusätzlich als Eduktstrom **S_{BE1}** in der Reaktivrektifikationskolonne **RR_{B}** eingesetzt wird.

10. Verfahren nach einem der Ansprüche 1 bis 9, wobei ein von **S_{AE1}** und **S_{BE1}** verschiedener Strom **S_{XE1}** umfassend ROH in mindestens eine der Kolonnen ausgewählt aus Rektifikationskolonne **RD₁,** Rektifikationskolonne **RD₂,** Reaktivrektifikationskolonne **RR_{A}** zugegeben wird und, wenn Schritt (a2) durchgeführt wird, alternativ oder zusätzlich in die Reaktivrektifikationskolonne **RR_{B}** zugegeben wird.

11. Verfahren nach einem der Ansprüche 1 bis 10, wobei R Methyl oder Ethyl ist.

12. Verfahren nach einem der Ansprüche 1 bis 11, wobei der Schritt (a2) durchgeführt wird.

13. Verfahren nach einem der Ansprüche 1 bis 12, wobei **p_{3A}** > **p₂** gilt, und in den Fällen, in denen Schritt (a2) durchgeführt wird, zusätzlich **p_{3B}** > **p₂** gilt.

14. Verfahren nach einem der Ansprüche 1 bis 13, wobei der Sumpfstrom **S_{RDS2}** Wasser und ROH umfasst.

15. Verfahren nach einem der Ansprüche 1 bis 14, welches kontinuierlich durchgeführt wird.

## Claims

1. Process for producing at least one alkali metal alkoxide of formula M_{A}OR, wherein R is a C₁ to C₆ hydrocarbon radical, and wherein M_{A} is selected from sodium, potassium, wherein:
(a1) a reactant stream S_{AE1} comprising ROH is reacted with a reactant stream S_{AE2} comprising M_{A}OH in countercurrent at a pressure p_{3A} and a temperature T_{3A} in a reactive rectification column RR_{A} to afford a crude product RP_{A} comprising M_{A}OR, water, ROH, M_{A}OH,
wherein a bottoms product stream S_{AP} comprising ROH and M_{A}OR is withdrawn at the lower end of RR_{A} and a vapour stream S_{AB} comprising water and ROH is withdrawn at the upper end of RR_{A},
(a2) and optionally, simultaneously with and spatially separate from step (a1), a reactant stream S_{BE1} comprising ROH is reacted with a reactant stream S_{BE2} comprising M_{B}OH in countercurrent at a pressure p_{3B} and a temperature T_{3B} in a reactive rectification column RR_{B} to afford a crude product RP_{B} comprising M_{B}OR, water, ROH, M_{B}OH, wherein M_{B} is selected from sodium, potassium,
wherein a bottoms product stream S_{BP} comprising ROH and M_{B}OR is withdrawn at the lower end of RR_{B} and a vapour stream S_{BB} comprising water and ROH is withdrawn at the upper end of RR_{B},
(b) the vapour stream S_{AB}, and, if step (a2) is performed, the vapour stream S_{BB}, is passed into a first rectification column RD₁ in admixture with S_{AB} or separately from S_{AB} to obtain a mixture G_{RD1} comprising water and ROH in the first rectification column RD₁,
(c) the mixture G_{RD1} in the first rectification column RD₁ at a pressure p₁ and a temperature T₁ is separated into an ROH-comprising vapour stream S_{RDB1} at the upper end of RD₁ and a bottoms stream S_{RDS1} comprising water and ROH at the lower end of RD₁,
(d) the bottoms stream S_{RDS1} is completely or partially passed into a second rectification column RD₂,
to obtain a mixture G_{RD2} comprising water and ROH in the second rectification column RD₂,
(e) the mixture G_{RD2} is separated at a pressure p₂ and a temperature T₂ into an ROH-comprising vapour stream S_{RDB2} at the top of RD₂ and a bottoms stream S_{RDS2} comprising water at the lower end of RD₂,
**characterized**
**in that** p₁ > p₂, p₁ > p_{3A}, and, in the cases in which step (a2) is performed, p₁ > p_{3B},
and **in that** (f) energy from S_{RDB1} is transferred to the mixture G_{RD2} in the second rectification column RD₂.

2. Process according to Claim 1, wherein, in step (f), energy is directly transferred from S_{RDB1} to G_{RD2}.

3. Process according to Claim 2, wherein at least one of the steps (α-i), (α-ii), (α-iii) is performed:
(α-i) energy from S_{RDB1} is transferred to a portion S_{RDS22} of the bottoms stream S_{RDS2} discharged from RD₂ and S_{RDS22} is then recycled into RD₂;
(α-ii) at least one stream S_{RDX2} distinct from S_{RDB2} and S_{RDS2} and comprising ROH and water is discharged from RD₂, energy is then transferred from S_{RDB1} to S_{RDX2} and S_{RDX2} is recycled into RD₂;
(α-iii) S_{RDB1} is passed through RD₂, thus transferring energy from S_{RDB1} to G_{RD2}.

4. Process according to Claim 1, wherein, in step (f), energy is indirectly transferred from S_{RDB1} to G_{RD2}.

5. Process according to Claim 4, wherein at least one of the steps (β-i), (β-ii), (β-iii) is performed:
(β-i) a portion S_{RDS22} of the bottoms stream S_{RDS2} discharged from RD₂ is recycled into the second rectification column RD₂, wherein energy is transferred from S_{RDB1} to at least one heat transfer medium Wᵢ₁ distinct from S_{RDS22} and then transferred from the at least one heat transfer medium Wᵢ₁ to S_{RDS22} and S_{RDS22} is then recycled into RD₂;
(β-ii) at least one stream S_{RDX2} distinct from S_{RDB2} and S_{RDS2} and comprising ROH and water is discharged from RD₂, and energy is transferred from S_{RDB1} to at least one heat transfer medium Wᵢᵢ₁ distinct from S_{RDX2} and then transferred from the at least one heat transfer medium Wᵢᵢ₁ to S_{RDX2}, and S_{RDX2} is then recycled into RD₂;
(β-iii) energy is transferred from S_{RDB1} to at least one heat transfer medium Wᵢᵢᵢ₁ distinct from G_{PD2}, and the at least one heat transfer medium Wᵢᵢᵢ₁ is then passed through RD₂, thus transferring energy from the at least one heat transfer medium Wᵢᵢᵢ₁ to G_{RD2}.

6. Process according to Claim 5, wherein each of Wᵢ₁, Wᵢᵢ₁, Wᵢᵢᵢ₁ is water.

7. Process according to any of Claims 3, 5 and 6, wherein S_{RDX2} is withdrawn below the vapour stream S_{RDB2} on RD₂.

8. Process according to any of Claims 1 to 7, wherein S_{RDB2} is at least partially employed as reactant stream S_{AE1} in the reactive rectification column RR_{A} and, if step (a2) is performed, alternatively or in addition as reactant stream S_{BE1} in the reactive rectification column RR_{B}.

9. Process according to any of Claims 1 to 8, wherein S_{RDB1} is at least partially employed as reactant stream S_{AE1} in the reactive rectification column RR_{A} and, if step (a2) is performed, alternatively or in addition as reactant stream S_{BE1} in the reactive rectification column RR_{B}.

10. Process according to any of Claims 1 to 9, wherein a stream S_{XE1} distinct from S_{AE1} and S_{BE1} and comprising ROH is added to at least one of the columns selected from rectification column RD₁, rectification column RD₂, reactive rectification column RR_{A} and, if step (a2) is performed, is alternatively or in addition added to the reactive rectification column RR_{B}.

11. Process according to any of Claims 1 to 10, wherein R is methyl or ethyl.

12. Process according to any of Claims 1 to 11, wherein step (a2) is performed.

13. Process according to any of Claims 1 to 12, wherein p_{3A} > p₂, and in addition, in the cases in which step (a2) is performed, p_{3B} > p₂.

14. Process according to any of Claims 1 to 13, wherein the bottoms stream S_{RDS2} comprises water and ROH.

15. Process according to any of Claims 1 to 14 which is performed continuously.

## Revendications

1. Procédé de préparation d'au moins un alcoolate de métal alcalin de formule M_{A}OR, dans laquelle R représente un radical hydrocarboné en C₁-C₆ et M_{A} est choisi parmi sodium, potassium, dans lequel :
(a1) un flux de départ S_{AE1} comprenant ROH est transformé avec un flux de départ S_{AE2} comprenant M_{A}OH à contre-courant dans une colonne de rectification réactive RR_{A} à une pression p_{3A} et à une température T_{3A} en un produit brut RP_{A} comprenant M_{A}OR, de l'eau, ROH, M_{A}OH,
où, en l'extrémité inférieure de RR_{A}, est prélevé un flux de produit de fond S_{AP} comprenant ROH et M_{A}OR et, en l'extrémité supérieure de RR_{A}, est prélevé un flux de vapeurs S_{AB} comprenant de l'eau et ROH,
(a2) et le cas échéant, simultanément avec l'étape (a1) et de manière spatialement séparée de celle-ci, un flux de départ S_{BE1} comprenant ROH est transformé avec un flux de départ S_{BE2} comprenant M_{B}OH à contre-courant dans une colonne de rectification réactive RR_{B} à une pression p_{3B} et à une température T_{3B} en un produit brut RP_{B} comprenant M_{B}OR, de l'eau, ROH, M_{B}OH, M_{B} étant choisi parmi sodium, potassium,
où, en l'extrémité inférieure de RR_{B}, est prélevé un flux de produit de fond S_{BP} comprenant ROH et M_{B}OR et, en l'extrémité supérieure de RR_{B}, est prélevé un flux de vapeurs S_{BB} comprenant de l'eau et ROH,
(b) le flux de vapeurs S_{AB}, et, lorsque l'étape (a2) est mise en œuvre, le flux de vapeurs S_{BB}, mélangé avec S_{AB} ou séparément de S_{AB}, est/sont conduit(s) dans une première colonne de rectification RD₁, suite à quoi un mélange G_{RD1} comprenant de l'eau et ROH est obtenu dans la première colonne de rectification RD₁,
(c) le mélange G_{RD1} dans la première colonne de rectification RD₁ est séparé à une pression p₁ et à une température T₁ en un flux de vapeurs S_{RDB1} comprenant ROH en l'extrémité supérieure de RD₁ et en un flux de fond S_{RDS1} comprenant de l'eau et ROH en l'extrémité inférieure de RD₁,
(d) le flux de fond S_{RDS1} est guidé, totalement ou partiellement, dans une deuxième colonne de rectification RD₂
suite à quoi un mélange G_{RD2} comprenant de l'eau et ROH est obtenu dans la deuxième colonne de rectification RD₂,
(e) le mélange G_{RD2} est séparé à une pression p₂ et à une température T₂ en un flux de vapeurs S_{RDB2} comprenant ROH en tête de RD₂ et en un flux de fond S_{RDS2} comprenant de l'eau en l'extrémité inférieure de RD₂,
**caractérisé**
**en ce que** p₁ > p₂, p₁ > p_{3A} sont d'application et, dans les cas où l'étape (a2) est mise en œuvre, p₁ > p_{3B} est d'application et en ce que (f) l'énergie de S_{RDB1} est transmise au mélange G_{RD2} dans la deuxième colonne de rectification RD₂.

2. Procédé selon la revendication 1, dans lequel, dans l'étape (f), l'énergie de S_{RDB1} est transmise directement à G_{RD2}.

3. Procédé selon la revendication 2, dans lequel au moins l'une des étapes (α-i), (α-ii), (α-iii) est mise en œuvre :
(α-i) l'énergie de S_{RDB1} est transmise à une partie S_{RDS22} du flux de fond S_{RDS2} évacué de RD₂ et S_{RDS22} est ensuite renvoyé dans RD₂ ;
(α-ii) au moins un flux S_{RDX2} différent de S_{RDB2} et S_{RDS2}, comprenant ROH et de l'eau, est évacué de RD₂, puis l'énergie de S_{RDB1} est transmise à S_{RDX2} et S_{RDX2} est renvoyé dans RD₂ ;
(α-iii) S_{RDB1} est guidé à travers RD₂, suite à quoi l'énergie est transmise de S_{RDB1} à G_{RD2}.

4. Procédé selon la revendication 1, dans lequel, dans l'étape (f), l'énergie de S_{RDB1} est transmise indirectement à G_{RD2}.

5. Procédé selon la revendication 4, dans lequel au moins l'une des étapes (β-i), (β-ii), (β-iii) est mise en œuvre :
(β-i) une partie S_{RDS22} du flux de fond S_{RDS2} évacué de RD₂ est renvoyé dans la deuxième colonne de rectification RD₂, l'énergie de S_{RDB1} étant transmise à au moins un agent caloporteur Wᵢ₁ différent de S_{RDS22} puis transmise dudit au moins un agent caloporteur Wᵢ₁ à S_{RDS22} et S_{RDS22} est ensuite renvoyé dans RD₂ ;
(β-ii) au moins un flux S_{RDX2} différent de S_{RDB2} et de S_{RDS2}, comprenant ROH et de l'eau est évacué de RD₂ et l'énergie est transmise de S_{RDB1} à au moins un agent caloporteur Wᵢᵢ₁ différent de S_{RDX2} puis dudit au moins un agent caloporteur Wᵢᵢ₁ à S_{RDX2} puis S_{RDX2} est renvoyé dans RD₂ ;
(β-iii) l'énergie est transmise de S_{RDB1} à au moins un agent caloporteur Wᵢᵢᵢ₁ différent de G_{RD2} et ledit au moins un agent caloporteur Wᵢᵢᵢ₁ est ensuite guidé à travers RD₂, suite à quoi l'énergie dudit au moins un agent caloporteur Wᵢᵢᵢ₁ est transmise à G_{RD2}.

6. Procédé selon la revendication 5, dans lequel Wᵢ₁, Wᵢᵢ₁, Wᵢᵢᵢ₁ sont à chaque fois de l'eau.

7. Procédé selon l'une des revendications 3, 5 et 6, dans lequel S_{RDX2} est prélevé au-dessous du flux de vapeurs S_{RDB2} de RD₂.

8. Procédé selon l'une des revendications 1 à 7, dans lequel S_{RDB2} est utilisé au moins partiellement comme flux de départ S_{AE1} dans la colonne de rectification réactive RR_{A} et, lorsque l'étape (a2) est mise en œuvre, en variante ou en plus comme flux de départ S_{BE1} dans la colonne de rectification réactive RR_{B}.

9. Procédé selon l'une des revendications 1 à 8, dans lequel S_{RDB1} est utilisé au moins partiellement comme flux de produit de départ S_{AE1} dans la colonne de rectification réactive RR_{A} et, lorsque l'étape (a2) est mise en œuvre, en variante ou en plus comme flux de produit de départ S_{BE1} dans la colonne de rectification réactive RR_{B}.

10. Procédé selon l'une des revendications 1 à 9, dans lequel un flux S_{XE1} différent de S_{AE1} et de S_{BE1} comprenant ROH est ajouté dans au moins l'une des colonnes choisies parmi la colonne de rectification RD₁, la colonne de rectification RD₂, la colonne de rectification réactive RR_{A} et, lorsque l'étape (a2) est mise en œuvre, en variante ou en plus dans la colonne de rectification réactive RR_{B}.

11. Procédé selon l'une des revendications 1 à 10, dans lequel R représente méthyle ou éthyle.

12. Procédé selon l'une des revendications 1 à 11, dans lequel l'étape (a2) est mise en œuvre.

13. Procédé selon l'une des revendications 1 à 12, dans lequel p_{3A} > p₂ est d'application et, dans les cas où l'étape (a2) est mise en œuvre, p_{3B} > p₂ est en plus d'application.

14. Procédé selon l'une des revendications 1 à 13, dans lequel le flux de fond S_{RDS2} comprend de l'eau et ROH.

15. Procédé selon l'une des revendications 1 à 14, qui est mis en œuvre en continu.
